# FASCICULE DE BREVET EUROPEEN

(11) **EP 2 806 958 B1**
(45) Date de publication et mention de la délivrance du brevet: **02.05.2018**
(21) Numéro de dépôt: 13704211.5
(22) Date de dépôt: 25.01.2013
(51) Int. Cl.: B01D 15/08, C02F 1/28, C02F 1/56, C07H 19/10

(54) **DÉCONTAMINATION PAR HYDROGELS D'ÉCHANTILLONS AQUEUX CONTENANT DES NANOPARTICULES**
HYDROGELBASIERTE DEKONTAMINIERUNG VON WÄSSRIGEN PROBEN MIT NANOPARTIKELN
HYDROGEL-BASED DECONTAMINATION OF AQUEOUS SAMPLES CONTAINING NANOPARTICLES

(30) Priorité: 25.01.2012 FR 1200233
(43) Date de publication de la demande: 03.12.2014
(73) Titulaire: Université de Bordeaux, 33000 Bordeaux (FR)
(72) Inventeur: BARTHÉLÉMY, Philippe, 33700 Mérignac (FR); THIÉRY, Alain, 84000 Avignon (FR); PATWA, Amit, 33076 Bordeaux Cedex (FR)
(74) Mandataire: Novagraaf Technologies
(86) Numéro de dépôt international: PCT/FR2013/050163
(87) Numéro de publication internationale: WO 2013/110902

(56) Documents cités:
- WO-A1-2005/116043
- GUILHEM GODEAU ET AL: "Glycosyl-Nucleoside Lipids as Low-Molecular-Weight Gelators +", LANGMUIR, vol. 25, no. 15, 4 août 2009 (2009-08-04), pages 8447-8450, XP055044901, ISSN: 0743-7463, DOI: 10.1021/la900140b
- GUILHEM GODEAU ET AL: "Glycosyl-nucleoside-lipid based supramolecular assembly as a nanostructured material with nucleic acid delivery capabilities", CHEMICAL COMMUNICATIONS, no. 34, 17 juillet 2009 (2009-07-17), page 5127, XP055044911, ISSN: 1359-7345, DOI: 10.1039/b906212b
- SISIR DEBNATH ET AL: "Dipeptide-Based Low-Molecular-Weight Efficient Organogelators and Their Application in Water Purification", CHEMISTRY - A EUROPEAN JOURNAL, vol. 14, no. 23, 8 août 2008 (2008-08-08), pages 6870-6881, XP055044918, ISSN: 0947-6539, DOI: 10.1002/chem.200800731
- GODEAU G ET AL: "Glycosyl-nucleoside fluorinated amphiphiles as components of nanostructured hydrogels", TETRAHEDRON LETTERS, ELSEVIER, AMSTERDAM, NL, vol. 51, no. 7, 17 février 2010 (2010-02-17), pages 1012-1015, XP026850864, ISSN: 0040-4039 [extrait le 2010-01-12] cité dans la demande
- L. S. STRIZHKO, S. I. LOLEIT, V. I. ZAKHAROVA: "Extracting noble metals from industrial solutions and waste waters with biosorbents", RUSSIAN JOURNAL OF NON-FERROUS METALS, vol. 50, no. 2, avril 2009 (2009-04), pages 118-122, XP002697268,
- MARÍA E. ROMERO-GONZÁLEZ ET AL: "Spectroscopic Studies of the Biosorption of Gold(III) by Dealginated Seaweed Waste", ENVIRONMENTAL SCIENCE & TECHNOLOGY, vol. 37, no. 18, 1 septembre 2003 (2003-09-01), pages 4163-4169, XP55063285, ISSN: 0013-936X, DOI: 10.1021/es020176w
- XINQING CHEN ET AL: "Precious metal recovery by selective adsorption using biosorbents", JOURNAL OF HAZARDOUS MATERIALS, ELSEVIER, AMSTERDAM, NL, vol. 186, no. 1, 22 novembre 2010 (2010-11-22), pages 902-910, XP028140252, ISSN: 0304-3894, DOI: 10.1016/J.JHAZMAT.2010.11.088 [extrait le 2010-11-30]
- QU R ET AL: "Adsorption of Au(III) from aqueous solution using cotton fiber/chitosan composite adsorbents", HYDROMETALLURGY, ELSEVIER SCIENTIFIC PUBLISHING CY. AMSTERDAM, NL, vol. 100, no. 1-2, 1 décembre 2009 (2009-12-01), pages 65-71, XP026769638, ISSN: 0304-386X, DOI: 10.1016/J.HYDROMET.2009.10.008 [extrait le 2009-10-25]
- NADEEM R ET AL: "Fourier Transform Infrared Spectroscopic characterization and optimization of Pb(II) biosorption by fish (Labeo rohita) scales", JOURNAL OF HAZARDOUS MATERIALS, ELSEVIER, AMSTERDAM, NL, vol. 156, no. 1-3, 15 août 2008 (2008-08-15), pages 64-73, XP022709245, ISSN: 0304-3894, DOI: 10.1016/J.JHAZMAT.2007.11.124 [extrait le 2007-12-15]
- SRIVIDYA K ET AL: "Biosorption of hexavalent chromium from aqueous solutions by Catla catla scales: Equilibrium and kinetics studies", CHEMICAL ENGINEERING JOURNAL, ELSEVIER SEQUOIA, LAUSANNE, CH, vol. 155, no. 3, 15 décembre 2009 (2009-12-15), pages 666-673, XP026769803, ISSN: 1385-8947, DOI: 10.1016/J.CEJ.2009.08.024 [extrait le 2009-08-31]
- YANG ZHANG ET AL: "Stability and Removal of Water Soluble CdTe Quantum Dots in Water", ENVIRONMENTAL SCIENCE & TECHNOLOGY, vol. 42, no. 1, 1 janvier 2008 (2008-01-01), pages 321-325, XP55063276, ISSN: 0013-936X, DOI: 10.1021/es0714991

## Description

### Domaine technique

La présente invention se rapporte à l'utilisation d'un système supramoléculaire pour soustraire des particules d'un milieu liquide les comprenant, dans laquelle ledit système supramoléculaire comprenant au moins une molécule à faible poids de formule (I) telle que définie dans la présente.

Elle se rapport également à un procédé de soustraction de particules d'un milieu liquide les contenant.

Elle trouve notamment des applications dans le domaine de la décontamination des eaux et des biotechnologies.

Dans la description ci-dessous, les références entre crochets ([]) renvoient à la liste des références présentée à la fin des exemples.

### Etat de la technique

De nombreuses recherches ont récemment été effectuées dans le domaine de la décontamination des eaux résiduaires, des eaux usées, des eaux potables et de manière générale de toutes solutions aqueuses utilisées notamment pour l'alimentation ou pour l'industrie.

Actuellement, le traitement des eaux contaminées consiste en l'utilisation de procédés physiques pour éliminer des éléments solides ou particulaires, de traitements physico-chimiques pour éliminer les matières particulaires décantables ou de traitements biologiques pour éliminer les matières organiques.

Ainsi, de nombreuses techniques ont été mises au point pour détruire ou éliminer des matériaux indésirables ou toxiques contenus dans des eaux souillées. Il s'agit, par exemple, de l'exposition des eaux aux ultraviolets (UV), de l'utilisation d'électrodes et d'un courant électrique pour capter des molécules chargées, de l'ajout d'agent coagulants, de floculants ou de chlore, de l'utilisation de microorganismes, etc.

Le charbon actif peut également être utilisé, mais les méthodes utilisant ce type de matériau se révèlent coûteuses et requièrent une maintenance et une gestion importante.

L'utilisation d'ozone (O₃) constitue également une méthode de décontamination principalement pour l'oxydation des matières organiques. Cependant cette méthode nécessite des installations complexes, utilisées pour traiter de grands volumes. Elle ne convient donc pas à la décontamination de faibles volumes.

Il s'avère par conséquent que les méthodes actuelles de décontaminations sont peu ou pas adaptées à décontaminer des milieux liquides pollués par des particules, notamment des particules de faible taille, par exemple des nanoparticules. En outre, ces méthodes ne sont pas forcément adaptées à (pour) décontaminer un milieu liquide aussi bien à grande échelle qu'à faibles volumes.

Les procédés de nanofiltration et d'osmose inverse sont les méthodes actuellement employées pour éliminer les nanoparticules des milieux liquides. Toutefois, ces méthodes impliquent des dispositifs et des mises en oeuvre très couteux, et nécessitent une forte maintenance.

Par ailleurs, de nombreuses préoccupations sanitaires relatives à une possible toxicité humaine, animale ou environnementale des nanoparticules ont vu le jour, en particulier suite à l'évaluation de l'Afssaps, publiée le 14 juin 2011, concernant les nanoparticules en cosmétique.

Il est donc crucial de mettre au point de nouvelles méthodes de traitement des milieux liquides permettant de résoudre ces problèmes de l'art antérieur, notamment de fournir des moyens permettant de débarrasser les milieux liquides des particules, en particulier des nanoparticules, qu'ils pourraient contenir. Il est également crucial de mettre au point un procédé de décontamination de milieu liquide contenant des particules, en particulier des nanoparticules, permettant de réduire les coûts et d'améliorer la décontamination par rapport aux méthodes de l'art antérieur.

### Exposé de l'invention

La présente invention répond précisément à ces besoins en fournissant un moyen permettant de décontaminer un milieu liquide contenant des particules indésirables.

Ainsi, la présente invention a notamment pour objet l'utilisation d'un système supramoléculaire pour soustraire des nanoparticules d'un milieu liquide aqueux les comprenant, dans laquelle ledit système supramoléculaire comprenant au moins une molécule à faible poids moléculaire de formule (I) définie ci-dessous.

Les inventeurs sont en effet les tous premiers à avoir constaté que des molécules à faible poids moléculaire de formule (I), définie ci-dessous, permettent de soustraire des particules d'un milieu liquide les comprenant en piégeant ces dernières par un système supramoléculaire séparable dudit milieu liquide. Des molécules structurellement similaires sont connues de la publication G.GODEAU ET AL: "Glycosyl-nucleoside-lipid based supramolecular assembly as a nanostructured material with nucleic acid delivery capabilities", CHEMICAL COMMUNICATIONS (34), p.5127. Notamment y sont décrits des assemblages supramoléculaires de lipides glycosyl-nucléosides (LNG) pour délivrer sélectivement des acides nucléiques emprisonnés au sein du gel formé par ces assemblages. La présente invention a également pour objet un procédé de soustraction de nanoparticules d'un milieu liquide aqueux les contenant, comprenant une étape (a) d'ajout d'un système supramoléculaire dans ledit milieu liquide à une température comprise entre 2 et 95°C, dans lequel le système supramoléculaire comprend au moins une molécule à faible poids moléculaire de formule (I), définie ci-dessous, ledit système supramoléculaire formant un gel au contact du milieu liquide aqueux, ledit gel captant les nanoparticules contenues dans ledit milieu liquide aqueux, et une étape (b) de séparation dudit milieu liquide aqueux et dudit gel ayant capté lesdites nanoparticules.

Selon l'invention, lorsque la température du milieu liquide aqueux obtenu à l'étape (a) est inférieure à 50°C, le procédé peut comprendre en outre avant l'étape (b), les étapes intermédiaires suivantes:
(a1) chauffage du milieu obtenu à l'étape (a) à une température comprise entre 50 et 95°C, et
(a2) refroidissement du milieu obtenu à l'étape (a1) à une température comprise entre 2 et 50°C.

Ces étapes (a1) et (a2) ne sont pas obligatoires. Elles permettent cependant d'augmenter très fortement la vitesse de formation du gel et donc la décontamination du milieu liquide.

Dans la présente, on entend par « soustraire des particules d'un milieu liquide les comprenant » ou « soustraction de particules d'un milieu liquide les contenant », le fait de piéger, ou capturer, des particules présentes dans un milieu liquide puis de les retirer dudit milieu. Selon l'invention, les particules sont piégées ou capturées par le système supramoléculaire, puis sont retirées du milieu liquide en séparant le gel formé par le système supramoléculaire du milieu liquide. Il peut s'agir par exemple de décontaminer un milieu liquide comprenant des particules ou d'une décontamination d'un milieu liquide comprenant des particules.

Dans la présente, on entend par « particule » un agrégat résultant de l'association de molécules organiques et/ou inorganiques via des liaisons faibles, (telles que les liaisons van der Waals ou les liaisons hydrogènes) ou bien de liaisons fortes (telles que les liaisons covalentes, ou les liaisons ioniques). Par conséquent, les particules ne possèdent pas de masse moléculaire.

Ainsi, selon l'invention, le terme particule exclut des molécules en solution, par exemple des colorants ou des oligonucléotides en solution, puisque ces dernières possèdent une masse moléculaire.

Dans la présente, on entend par « nanoparticules », des particules de taille nanométrique (ou particule outre-fine), selon la norme ISO TS/27687, comme étant un assemblage de molécules dont au moins une des dimensions se situe à l'échelle nanométrique. Par exemple, une « nanoparticule » selon l'invention peut être définie selon la norme ISO précitée comme étant un nano-objet dont les trois dimensions sont à l'échelle nanométrique, c'est-à-dire des particules dont le diamètre nominal est inférieur à 100 nm environ, de préférence compris entre 0.5 nm et 100 nm.

La présente invention est particulièrement efficace pour décontaminer un milieu liquide contaminé avec des nanoparticules par exemple des particules dont la taille est comprise entre 0,5 nm et 5 µm, par exemple entre 0,5 nm et 1 µm, par exemple entre 0,5 nm et 500 nm, par exemple entre 1 et 50 nm, par exemple entre 5 et 20 nanomètre.

De manière avantageuse, les particules ont une taille comprise entre 1 nm et 500 nm, par exemple entre 1 nm et 400 nm, par exemple entre 5 nm et 300 nm, par exemple entre 5 nm et 200 nm. les particules sont des nanoparticules, c'est-à-dire des particules dont la taille est comprise entre 5 nm et 150 nm, par exemple entre 5 nm et 100 nm.

A titre d'exemple de particules, ont peut citer les nanocristaux semiconducteurs fluorescents, les particules d'or, les particules d'argent, les particules de TiO₂, les particules de CeO, les particules de ZnO. Il peut s'agir par exemple de toutes particules ou toutes nanoparticules présentes dans des produits cosmétiques tels que des crèmes solaires, dans des produits d'entretien tels que des déodorants, dans des produits de rénovation tels que les peintures, etc.

Les nanocristaux semiconducteurs fluorescents (« QDs », « points quantiques » ou « quantum dots » en anglais) sont des nanoparticules inorganiques de taille nanométrique, d'environ 10 nm, ayant les propriétés optiques uniques. Solubles dans l'eau, ils s'avèrent utiles comme alternative aux fluorophores organiques dans certaines applications biomédicales et biotechnologiques. En conséquence, les déchets liquides rejetés par les industries chimiques et des instituts de recherche peuvent souvent être contaminés par des QDs encapsulés. La présente invention fournie justement des moyens pour piéger ou supprimer les QDs présents dans un milieu liquide, afin de décontaminer ce milieu liquide de ces nanoparticules.

Les nanoparticules d'or (AuNPs) sont des composés inorganiques de taille nanométrique, inférieure à 100 nm, présentant des propriétés optiques, électroniques et moléculaires de reconnaissance, uniques, utiles dans une grande variété de domaines, notamment la microscopie électronique, l'électronique, la nanotechnologie et science des matériaux. En conséquence, les déchets liquides rejetés par les industries chimiques et des instituts de recherche peuvent être pollués par des AuNPs. La présente invention fournie justement des moyens pour piéger ou supprimer les AuNPs présents dans un milieu liquide, afin de décontaminer ce milieu liquide de ces nanoparticules.

Un milieu liquide choisi dans le groupe comprenant un milieu aqueux, un solvant organique, une émulsion et un milieu polyphasique est décrit.

Dans la présente, on entend par « milieu aqueux », tout mélange comprenant au moins un liquide et dont le solvant est de l'eau.

Dans la présente, on entend par « solvant organique » tout solvant comprenant des composés organiques. Par exemple, le milieu liquide peut être un solvant organique choisi dans le groupe comprenant un hydrocarbure, un solvant oxygéné et un solvant halogéné.

Par exemple, l'hydrocarbure peut être un hydrocarbure aliphatique, par exemple un alcane ou un alcène, ou un hydrocarbure aromatique, par exemple le benzène, le toluène ou le xylène.

Le solvant oxygéné peuvent être, par exemple, choisi dans le groupe comprenant l'éthanol, le méthanol, l'acétone, d'acide acétique, l'acétate d'éthyle, un éther et un éther de glycol.

Le solvant halogéné peut être, par exemple, choisi dans le groupe comprenant du perchloroéthylène, du trichloréthylène, du dichlorométhane, du chloroforme et du tétrachlorométhane.

Dans la présente, on entend par « milieu polyphasique », un milieu comprenant au moins deux substances liquides non miscibles. Le milieu polyphasique peut être, par exemple, un milieu diphasique, c'est-à-dire comprenant deux phases, par exemple il peut s'agir d'une émulsion.

Dans la présente, on entend par « émulsion », tout mélange, macroscopiquement homogène mais microscopiquement hétérogène, de deux substances liquides non miscibles. Par exemple, il peut s'agir d'une émulsion du type eau dans l'huile ou d'une émulsion du type huile dans l'eau.

Le milieu liquide conforme à l'invention est un milieu aqueux.

Dans la présente description, les exemples de « base hétérocyclique » comprennent, un groupe hétérocyclique de 5 à 14 chaînons, de préférence, de 5 à 10 chaînons, (monocyclique, bicyclique ou tricyclique) ayant, en tant que chaînon hormis les atomes de carbone 1 à 4 hétéroatomes choisis dans le groupe consistant en un atome d'azote, un atome de soufre et un atome d'oxygène.

Dans la présente description, les exemples de « chaîne alkyle, linéaire ou ramifiée, en C₁-C₅ », comprennent le méthyle, éthyle, propyle, isopropyle, butyle, isobutyle, sec-butyle, tert-butyle, pentyle, isopentyle, néopentyle, tert-pentyle.

Dans la présente description, les exemples de « chaîne alkyle, linéaire ou ramifiée, en C₂-C₃₀ », comprennent l'éthyle, propyle, isopropyle, butyle, isobutyle, sec-butyle, tert-butyle, pentyle, isopentyle, néopentyle, tert-pentyle, hexyle, heptyle, octyle, nonyle, décyle, undecyle, dodécyle, triskaidecyle, tetradécyle, pentadecyle, hexadecyle, heptadecyle, octadecyle enneadecyle, eicosanyle, triacontanyle ou insaturé de types oléique, linoléique, linoléique.

Dans la présente description, on entend par « hétéroaryle comprenant 1 à 4 atomes d'azote », un aryle comprenant de 1 à 4 hétéroatomes, notamment choisi dans le groupe comprenant le soufre, l'oxygène, l'azote, le bore. A titre d'exemple d'hétéroaryle comprenant 1 à 4 atomes d'azote on peut citer les composés suivants: 1,2-diazole, 1,3-diazole, 1,4-diazole, 1,2,3-triazole, 1,2,4-triazole, 1,3,4-triazole, les tetrazole.

Dans la présente description, les exemples de « chaîne hydroxyalkyle » comprennent des chaînes saturées et insaturées. A titre d'exemple de chaines hydroxyalkyle saturées en C₁-C₁₉, on peut citer les chaines hydroxyoctyle, hydroxynonyle, hydroxydecyle, hydroxydodecyle, hydroxytetradécyle, hydroxytetradécyle, hydroxypentadecyle, hydroxyhexadecyle, hydroxyheptadecyle, hydroxyoctadecyle hydroxyenneadecyle.

Dans la présente description, les exemples de « chaine acyle en C₂-C₃₀ » comprennent l'éthyle, propyle, isopropyle, butyle, isobutyle, sec-butyle, tert-butyle, pentyle, isopentyle, néopentyle, tert-pentyle, hexyle, heptyle, octyle, nonyle, décyle, undecyle, dodécyle, triskaidecyle, tetradécyle, pentadecyle, hexadecyle, heptadecyle, octadecyle enneadecyle, eicosanyle, triacontanyle ou unsaturé de types oléique, linoléique, linoléique.

Dans la présente description, les exemples de « chaine hydrocarbonée en C₂-C₃₀ » peuvent être saturées ou insaturées et peuvent être ramifiés ou non. A titre d'exemple de chaine hydroalkyle saturées en C₂-C₃₀, on peut citer les chaines octyle, nonyle, decyle, dodecyle, myristique, palmitique et stéarique. A titre d'exemple de chaine hydroalkyle insaturées en C₂-C₃₀, on peut citer les chaines oléique, linoléique, linoléique et arachidonique.

Dans la présente description, les exemples de « hydrocarbonée en C₁-C₁₉ » peuvent être saturées ou insaturées et peuvent être ramifiés ou non. A titre d'exemple de chaines hydroalkyle saturées en C₁-C₁₉, on peut citer les chaines octyle, nonyle, decyle, dodecyle, myristique, palmitique et stéarique. A titre d'exemple de chaine hydroalkyle insaturées en C₁-C₁₉, on peut citer les chaines oléique, linoléique, linoléique

Dans la présente description, les exemples de « chaine fluorocarbonée» comprennent de 1 à 30 atomes de carbone et peuvent être choisies dans le groupe comprenant le trifluoromethyle, le pentafluoroethyle, l'heptafluoropropyle, le nonafluorobutyle, l'undecafluoropentyle, le perfluorohexyle, le perfluorooctyle, le perfluorodecyle, le perfluoroundecyle, le perfluorododecyle, le perfluoroundecyle et le perfluorododecyle.

Dans la présente description, les exemples de « chaine hydrofluorocarbonée » comprennent de 1 à 30 atomes de carbone et peuvent être choisies dans le groupe comprenant 1*H*,1*H*,2*H*,2*H-*perfluoroheptyle, 1*H*,1*H*,2*H*,2*H*-perfluorooctyle, 1*H,*1*H,*2*H,*2*H-*perfluorononyle, 1*H,*1*H*,2*H*,2*H*-perfluorodecyle, 1*H,*1*H,*2*H,*2*H-*perfluoroundecyle, 1*H,*1*H*,2*H*,2*H*-perfluorododecyle, 7*H*,7*H*, 7*H*,6*H*,6*H-*perfluoroheptyle, 8*H*,8*H*,8*H*,7*H*,7*H*-perfluorooctyle, 9*H*,9*H*,9*H*,8*H*,8*H-*perfluorononyle, 10*H*,10*H*,10*H*,9*H*,9*H*-perfluorodecyle, 11*H*,11*H*,11*H*,10*H*,10*H*-perfluoroundecyle, 12*H*,12*H*,12*H*,11*H,* 11*H* perfluorododecyle, 1*H*,1*H,*3*H,*3*H,*5*H,*5*H,*7*H,*7*H,*9*H,*9*H*11*H*,11*H-*perfluorododecyle et *2H*,2*H*,4*H*,4*H*,6*H*,6*H*,8*H*,8*H*,10*H*,10*H*,12*H*,12*H-*perfluorododecyle.

Dans la présente description, les exemples de « monosaccharide » comprennent le dihydroxyacétone, le glycéraldéhyde, l'erythrulose, l'érythrose, le thréose, le ribulose, le xylulose, l'arabinose, le lyxose, le ribose, le xylose, le désoxyribose, le fructose, le psicose, le sorbose, le tagatose, l'allose, l'altrose, le galactose, le glucose, le gulose, l'idose, le mannose, le talose, le fucose, le fuculose, le pneumose, le quinovose, le rhamnose, le glucoheptose, l'idoheptulose, le mannoheptulose, le sédoheptulose, le taloheptulose. De préférence, le monosaccharide est un glucose ou un galactose.

Dans la présente description, les exemples de « polysaccharide » comprennent le cellobiose, le gentiobiose, l'inulobiose, l'isomaltose, l'isomaltulose, le kojibiose, le lactose, le lactulose, le laminaribiose, le leucrose, le maltose, le maltulose, le mélibiose, le nigerose, le robinose, le rutinose, le saccharose, le sophorose, le tréhalose, le tréhalulose, le turanose, l'erlose , le fucosyllactose, le gentianose, l'inulotriose, le 1-kestose, le 6-kestose, le maltotriose, le mannotriose, le mélézitose, le néokestose, le panose, le raffinose, le rhamninose.

Dans la présente au moins une molécule à faible poids moléculaire est de formule (I) ci-dessous est décrite: 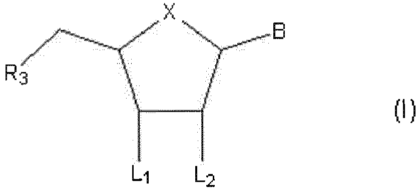 dans laquelle:
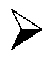 X représente un atome d'oxygène, un atome de soufre ou un groupe méthylène;
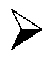 B représente une base purique ou pyrimidique, ou une base hétérocylique, mono-cyclique ou bi-cyclique, non naturelle dont chaque cycle comporte 4 à 7 chaînons, éventuellement substituée par un groupement R₃' tel que défini ci-dessous;
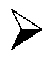 les substituants L₁ et L₂:
   ο sont identiques ou différents et représentent:
      (i) un atome d'hydrogène,
      (ii) un groupe hydroxy,
      (iii) un groupe hétéroaryle comprenant 1 à 4 atomes d'azote, non substitué ou substitué par une chaîne hydrocarbonée, saturée ou insaturée, linéaire ou ramifiée en C₂-C₃₀,
      (iv) un mono- ou un polysaccharide, ou
      (v) un groupement choisi parmi un groupe oxycarbonyl -OC(O)-, un groupe thiocarbamate -O-C(S)-NH-, un groupe carbonate -O-C(O)-O-, un groupe carbamate -O-C(O)-NH-, un groupe éther -O-, un groupe phosphate et un groupe phosphonate, sachant que ledit groupement L₁ est substitué par un groupement R₁ et ledit groupement L₂ est substitué par un groupement R₂, où R₁ et R₂ identiques ou différents, représentent:
         - une chaîne hydrocarbonée, linéaire ou ramifiée, en C₂-C₃₀, de préférence en C₆-C₂₅, plus préférentiellement en C₈-C₂₅, saturée ou insaturée, partiellement fluorée ou perfluorée, non substituée ou substituée sur le carbone d'extrémité de chaîne par un atome de fluor ou par un ester ou un éther benzylique ou naphtylique,
         - un radical acyle en C₂-C₃₀, ou
         - un groupe acylglycérol, sphingosine ou céramide,
      ou
   ο forment un groupement cétal de formule (II) ci-dessous: 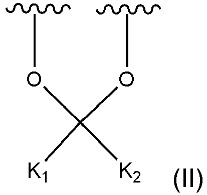 formule (II) dans laquelle K₁ et K₂ sont identiques ou différents et représentent une chaîne hydrocarbonée, saturée ou insaturée, en C₁-C₁₉,
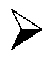 R₃ et R₃'
   - représentent indépendamment l'un de l'autre:
      (i) un groupement hydroxy, amino, phosphate, phosphonate, phospholcholine, O-alkyl phosphocholine, thiophosphate, phosphonium, -[NH₂-R₄]⁺, -[NHR₄R₅]⁺ ou -[NR₄R₅R₆]⁺ dans lequel R₄, R₅ et R₆, indépendamment l'un de l'autre, sont identiques ou différents, et représentent (a) un atome d'hydrogène, (b) une chaîne alkyle, linéaire ou ramifiée, en C₁-C₅, ou (c) une chaîne hydroxyalkyle, linéaire ou ramifié, en C₁-C₅,
      (ii) une chaîne alkyle linéaire ou ramifiée en C₂-C₃₀, éventuellement substituée par au moins un groupe hydroxy,
      (iii) un groupement cyclodextrine,
      (iv) un groupe -(CH₂)ₙ-V-R₈, dans lequel V représente un groupe -O-, -S-, ou -NH-, R₈ représente un alkyle en C₂-C₃₀, et n est un entier de 1 à 50,
      (v) un groupe -V-C(O)-R₈, dans lequel V représente un groupe -O-, -S-, ou -NH-, R₈ représente un alkyle en C₂-C₃₀,
         ou
      (vi) un groupe hétéroaryle renfermant de 1 à 4 atomes d'azote, ledit groupe hétéroaryle étant non substitué ou substitué par un alkyle en C₂-C₃₀, ou par un groupe -(CH₂)ₘ-O-(CH₂)ₚ-R₉, ou un groupe -(CH₂)₀₋₁-Y-C(=O)-R", ou un mono- ou polysaccharide, ou un groupe : 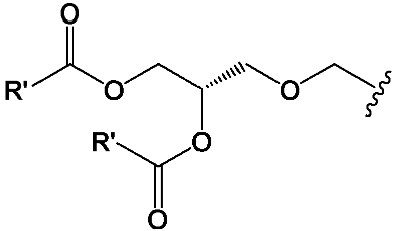 ou un groupe : 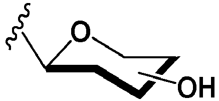 dans lesquels:
         m est un entier de 1 à 6,
         p est un entier de 0 à 10 et
         Rg représente un groupement alkyl en C₁ à C₁₀ , ou un groupe cétal cyclique renfermant 5 à 7 atomes de carbone, ledit groupe cétal cyclique étant non substitué ou substitué par au moins un alkyle linéaire ou ramifié en C₂-C₃₀, un groupement stérol, un diacyl glycérol, une chaîne hydrofluorocarbonée ou au moins un mono- ou polysaccharide,
         Y est un atome d'oxygène, un groupe NH, ou un atome de soufre,
         et R" est une chaîne hydrocarbonée ou une chaîne fluorocarbonée R' est une chaîne hydrocarbonée, ou
   - sont liés indépendamment l'un de l'autre par liaison covalente à un autre substituant R₃ ou R₃', identique ou différent, d'un autre composé de formule (I), identique ou différent, pour former un composé sous forme de dimère.

Dans la présente, les bases puriques ou pyrimidiques, non naturelles peuvent englober la base : 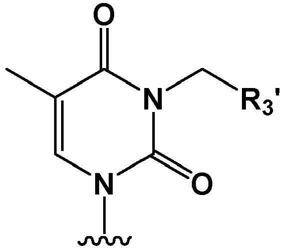 dans laquelle :
R₃' est tel que défini précédemment.

Dans la présente, le groupe phosphocholine a la formule suivante :

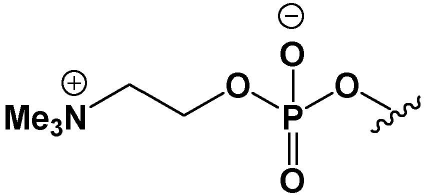

Dans la présente, le groupement stérol tel que défini précédemment peut être par exemple un radical cholestéryl.

Par exemple, le composé organique peut être un polymère.

Dans la présente au moins une molécule à faible poids moléculaire est de formule (I) ci-dessous est décrite: 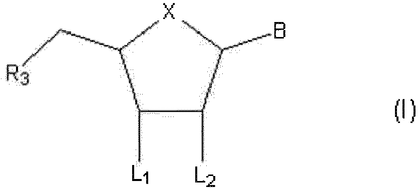 dans laquelle:
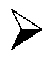 X représente un atome d'oxygène, un atome de soufre ou un groupe méthylène;
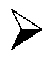 B représente une base purique ou pyrimidique, ou une base hétérocylique, mono-cyclique ou bi-cyclique, non naturelle dont chaque cycle comporte 4 à 7 chaînons, éventuellement substituée par un groupement R₃ tel que défini ci-dessous;
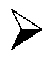 les substituants L₁ et L₂:
   ο sont identiques ou différents et représentent:
      (i) un atome d'hydrogène,
      (ii) un groupe hydroxy,
      (iii) un groupe hétéroaryle comprenant 1 à 4 atomes d'azote, non substitué ou substitué par une chaîne hydrocarbonée, saturée ou insaturée, linéaire ou ramifiée en C₂-C₃₀,
      (iv) un mono- ou un polysaccharide, ou
      (v) un groupement choisi parmi un groupe oxycarbonyl -OC(O)-, un groupe thiocarbamate -O-C(S)-NH-, un groupe carbonate -O-C(O)-O-, un groupe carbamate -O-C(O)-NH-, un groupe éther -O-, un groupe phosphate et un groupe phosphonate, sachant que ledit groupement L₁ est substitué par un groupement R₁ et ledit groupement L₂ est substitué par un groupement R₂, où R₁ et R₂ identiques ou différents, représentent:
         - une chaîne hydrocarbonée, linéaire ou ramifiée, en C₂-C₃₀, de préférence en C₆-C₂₅, plus préférentiellement en C₈-C₂₅, saturée ou insaturée, partiellement fluorée ou perfluoré, non substituée ou substituée sur le carbone d'extrémité de chaîne par un atome de fluor ou par un ester ou un éther benzylique ou naphtylique,
         - un radical acyle en C₂-C₃₀, ou
         - un groupe acylglycérol, sphingosine ou céramide,
      ou
   ο forment un groupement cétal de formule (II) ci-dessous: 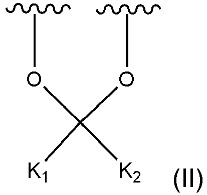 formule (II) dans laquelle K₁ et K₂ sont identiques ou différents et représentent une chaîne hydrocarbonée, saturée ou insaturée, en C₁-C₁₉,
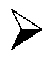 R₃
   - représente:
      (i) un groupement hydroxy, amino, phosphate, phosphonate, phospholcholine, O-alkyl phosphocholine, thiophosphate, phosphonium, -NH₂-R₄, -NHR₄R₅ ou -NR₄R₅R₆ dans lequel R₄, R₅ et R₆, indépendamment l'un de l'autre, sont identiques ou différents, et représentent (a) un atome d'hydrogène, (b) une chaîne alkyle, linéaire ou ramifiée, en C₁-C₅, ou (c) une chaine hydroxyalkyle, linéaire ou ramifié, en C₁-C₅,
      (ii) une chaîne alkyle linéaire ou ramifiée en C₂-C₃₀, éventuellement substituée par au moins un groupe hydroxy,
      (iii) un groupement cyclodextrine,
      (iv) un groupe -(CH₂)ₙ-V-R₈, dans lequel V représente un groupe - O-, -S-, ou -NH-, R₈ représente un alkyle en C₂-C₃₀ , et n est un entier de 1 à 50,
      (v) un groupe -V-C(O)-R₈, dans lequel V représente un groupe -O-, -S-, ou -NH-, R₈ représente un alkyle en C₂-C₃₀,
         ou
      (vi) un groupe hétéroaryle renfermant de 1 à 4 atomes d'azote, ledit groupe hétéroaryle étant non substitué ou substitué par un alkyle en C₂-C₃₀, ou par un groupe -(CH₂)ₘ-O-(CH₂)ₚ-R₉,
         dans lequel:
         m est un entier de 1 à 6,
         p est un entier de 0 à 10 et
         R₉ représente un groupe cétal cyclique renfermant 5 à 7 atomes de carbone, ledit groupe cétal cyclique étant non substitué ou substitué par au moins un alkyle linéaire ou ramifié en C₂-C₃₀, un groupement stérol, un diacyl glycérol, une chaine hydrofluorocarbonée ou au moins un mono- ou polysaccharide,
      ou
   - est lié par liaison covalente à un autre substituant R₃, identique ou différent, d'un autre composé de formule (I), identique ou différent, pour former un composé sous forme de dimère.

Selon l'invention l'au moins une molécule à faible poids moléculaire est de formule (I) ci-dessous : 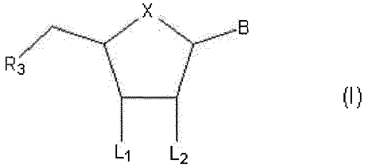 dans laquelle
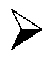 X représente un atome d'oxygène,
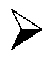 B représente une base purique ou pyrimidique naturelle ou non, éventuellement substituée par un groupement R₃' tel que défini ci-dessous;
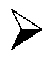 les substituants L₁ et L₂:
   ο sont identiques ou différents et représentent:
      (i) un atome d'hydrogène,
      (ii) un groupe hydroxy,
      (iii) un groupe hétéroaryle comprenant 1 à 4 atomes d'azote, non substitué ou substitué par une chaîne hydrocarbonée, saturée ou insaturée, linéaire ou ramifiée en C₂-C₃₀,
      (iv) un groupement choisi parmi un groupe oxycarbonyl -OC(O)-, un groupe thiocarbamate -O-C(S)-NH-, un groupe carbonate -O-C(O)-O-, un groupe carbamate -O-C(O)-NH-, un groupe éther -O-, un groupe phosphate et un groupe phosphonate, sachant que ledit groupement L₁ est substitué par un groupement R₁ et ledit groupement L₂ est substitué par un groupement R₂, où R₁ et R₂ identiques ou différents, représentent:
         - une chaîne hydrocarbonée, linéaire ou ramifiée, en C₂-C₃₀, de préférence en C₆-C₂₅, plus préférentiellement en C₈-C₂₅, saturée ou insaturée, partiellement fluorée ou perfluorée,
         - un radical acyle en C₂-C₃₀, ou
         - un groupe acylglycérol,
      ou
   ο forment un groupement cétal de formule (II) ci-dessous: 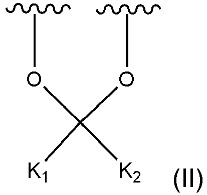 formule (II) dans laquelle K₁ et K₂ sont identiques ou différents et représentent une chaîne hydrocarbonée, saturée ou insaturée, en C₁-C₁₉,
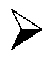 R₃ et R₃'
   - représentent indépendamment l'un de l'autre:
      (i) un groupement hydroxy, amino, phosphate, phosphonate, phosphocholine, O-alkyl phosphocholine, thiophosphate, phosphonium,
      (ii) une chaîne alkyle linéaire ou ramifiée en C₂-C₃₀, éventuellement substituée par au moins un groupe hydroxy,
      (iii) un groupe -(CH₂)ₙ-V-R₈, dans lequel V représente un groupe -O-, -S-, ou -NH-, R₈ représente un alkyle en C₂-C₃₀, et n est un entier de 1 à 50,
      (iv) un groupe -V-C(O)-R₈, dans lequel V représente un groupe -O-, -S-, ou -NH-, R₈ représente un alkyle en C₂-C₃₀,
         ou
      (v) un groupe hétéroaryle renfermant de 1 à 4 atomes d'azote, ledit groupe hétéroaryle étant non substitué ou substitué par un alkyle en C₂-C₃₀, ou par un groupe -(CH₂)ₘ-O-(CH₂)ₚ-R₉, ou un groupe -(CH₂)₀₋₁-Y-C(=O)-R", ou un mono- ou polysaccharide, ou un groupe : 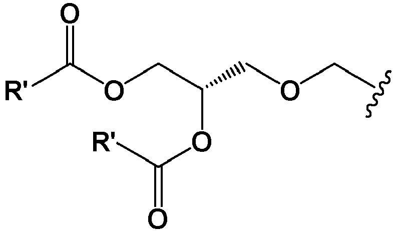 ou un groupe : 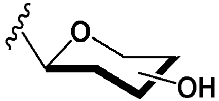 dans lesquels:
         m est un entier de 1 à 6,
         p est un entier de 0 à 10 et
         Rg représente un groupement alkyl en C₁ à C₁₀, ou un groupe cétal cyclique renfermant 5 à 7 atomes de carbone, ledit groupe cétal cyclique étant non substitué ou substitué par au moins un alkyle linéaire ou ramifié en C₂-C₃₀, un groupement stérol, un diacyl glycérol, une chaîne hydrofluorocarbonée ou au moins un mono- ou polysaccharide,
         Y est un atome d'oxygène, un groupe NH, ou un atome de soufre,
         et R" est une chaîne hydrocarbonée ou une chaîne fluorocarbonée, R' est une chaîne hydrocarbonée,
      ou
   - sont liés indépendamment liés l'un de l'autre par liaison covalente à un autre substituant R₃ ou R₃', identique ou différent, d'un autre composé de formule (I), identique ou différent, pour former un composé sous forme de dimère.

Par exemple, selon l'invention, dans la formule (I) ci-dessus,
X représente un atome d'oxygène,
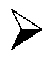 B représente une base thymine, adénine, guanine, cytosine, 6-méthoxypurine, hypoxanthine ou une base purique ou pyrimidique non naturelle qui peut englober: 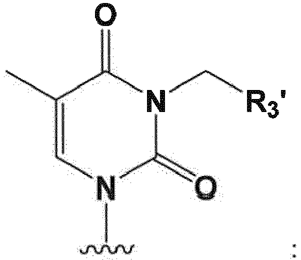 dans laquelle :
   R₃' est tel que défini ci-dessous,
   et/ou
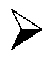 L₁ représente un groupe hydroxy, L₂ représente un atome d'hydrogène, ou L₁ et L₂ forment ensemble un groupement cétal de formule (II) ci-dessous: 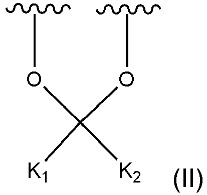 dans laquelle K₁ et K₂ sont identiques ou différents et représentent une chaîne hydrocarbonée, saturée ou insaturée, en C₁-C₁₉ et/ou
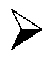 R₃ et R₃'
   - représentent:
      (i) un groupement hydroxy, amino, phosphate, phosphonate, phosphocholine, O-alkyl phosphocholine, thiophosphate ou phosphonium,
      (ii) une chaîne alkyle linéaire ou ramifiée en C₂-C₃₀, éventuellement substituée par au moins un groupe hydroxy,
      (iii) un groupe -(CH₂)ₙ-V-R₈, dans lequel V représente un groupe -O-, -S-, ou -NH-, R₈ représente un alkyle en C₂-C₃₀, et n est un entier 1 à 50,
      (iv) un groupe -V-C(O)-R₈, dans lequel V représente un groupe -O-, -S-, ou -NH-, R₈ représente un alkyle en C₂-C₃₀, ou
      (v) un groupe hétéroaryle renfermant de 1 à 4 atomes d'azote, ledit groupe hétéroaryle étant non substitué ou substitué par un alkyle en C₂-C₃₀, ou par un groupe (CH₂)ₘ-O-(CH₂)ₚ-R₉, ou un groupe-(CH₂)₀₋₁-Y-C(=O)-R", ou un mono- ou polysaccharide, ou un groupe : 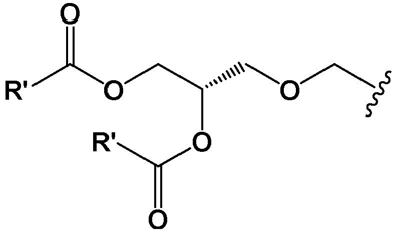 ou un groupe: 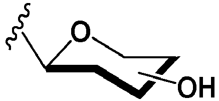 dans lesquels:
         m est un entier de 1 à 6,
         p est un entier de 0 à 10 et
         Rg représente un groupement alkyl en C₁ à C₁₀, ou un groupe cétal cyclique renfermant 5 à 7 atomes de carbone, ledit groupe cétal cyclique étant non substitué ou substitué par au moins un alkyle linéaire ou ramifié en C₂-C₃₀, un groupement stérol, un diacyl glycérol, une chaîne hydrofluorocarbonée ou au moins un mono- ou polysaccharide,
         Y est un atome d'oxygène, un groupe NH, ou un atome de soufre,
         et R" est une chaîne hydrocarbonée ou une chaîne fluorocarbonée, R' est une chaîne hydrocarbonée, ou
   - sont liés indépendamment l'un de l'autre par liaison covalente à un autre substituant R₃ ou R₃', identique ou différent, d'un autre composé de formule (I), identique ou différent, pour former un composé sous forme de dimère.

Selon l'invention, dans la formule (I) ci-dessus :
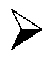 X représente un atome d'oxygène,
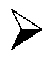 B représente une base pyrimidique non naturelle substituée par un groupe hétéroaryle renfermant 3 atomes d'azote, ledit groupe hétéroaryle étant substitué par un groupe: 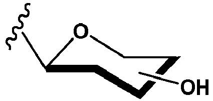
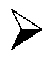 les substituants L₁ et L₂ sont identiques ou différents et représentent:
   (i) un atome d'hydrogène,
   (ii) un groupe hydroxy,
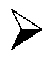 R₃
   - représente un groupe hétéroaryle comprenant 3 atomes d'azote, ledit groupe hétéroaryle étant substitué par un groupe : 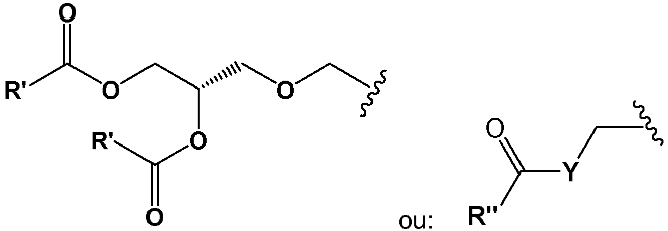 dans lesquels :
      R'est une chaîne hydrocarbonée,
      Y est un atome d'oxygène, un groupe NH, ou un atome de soufre,
      et R" est une chaîne hydrocarbonée ou une chaîne fluorocarbonée, ou
   - est lié à un autre substituant R₃, identique ou différent, d'un autre composé de formule (I), identique ou différent, pour former un composé sous forme de dimère.

Selon l'invention, dans la formule (I) ci-dessus,
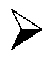 X représente un atome d'oxygène,
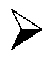 B représente une base purique ou pyrimidique;
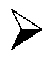 les substituants L₁ et L₂ forment un groupement cétal de formule (II) ci-dessous: 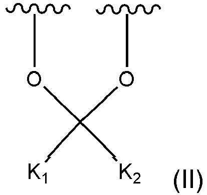 dans laquelle K₁ et K₂ sont identiques ou différents et représentent une chaîne hydrocarbonée, saturée ou insaturée, en C₁-C₁₉,
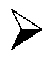 R₃
   - représente un groupement phosphocholine
      ou
   - est lié à un autre substituant R₃, identique ou différent, d'un autre composé de formule (I), identique ou différent, pour former un composé sous forme de dimère.

Selon l'invention, X représente un atome d'oxygène,

Par exemple, dans la formule (I) ci-dessus, la base purique, pyrimidique ou hétérocyclique non naturelle peut être substituée par au moins un substituant choisi parmi un halogène et un groupe amino, carboxy, carbonyl, carbonylamino, hydroxy, azido, cyano, alkyle, cycloalkyl, perfluoroalkyl, alkyloxy, oxycarbonyl, vinyl, ethynyl, propynyl ou acyl.

Avantageusement, une molécule à faible poids moléculaire conforme à l'invention a une masse moléculaire comprise entre 400 Da et 5000 kDa.

Par exemple, selon l'invention, l'au moins une molécule à faible poids moléculaire peut être un glycosyl-nucléoside amphiphile, par exemple un glycosyl-nucléoside fluoré amphiphile (« GNF »).

Par exemple, l'au moins une molécule à faible poids moléculaire peut être choisie dans:
- le groupe défini par la formule (III) ci-dessous, dans lesquels R' est une chaîne hydrocarbonée: 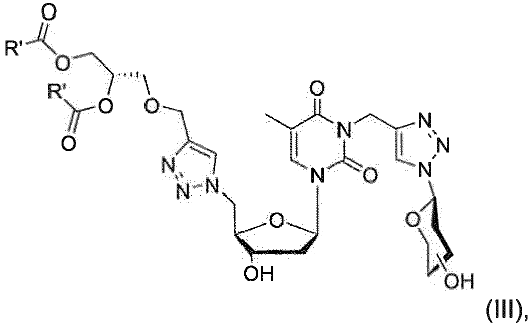
- le groupe défini par la formule (IV) ci-dessous, dans lequel Y est un atome d'oxygène, un groupe NH ou un atome de soufre, et R" est une chaîne hydrocarbonée ou une chaîne flurocarbonée: 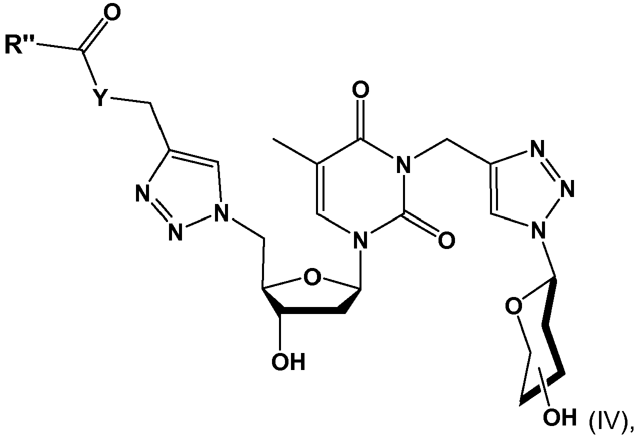
- le groupe défini par la formule (V) ci-dessous, dans lequel n est un nombre entier compris entre 0 et 19: 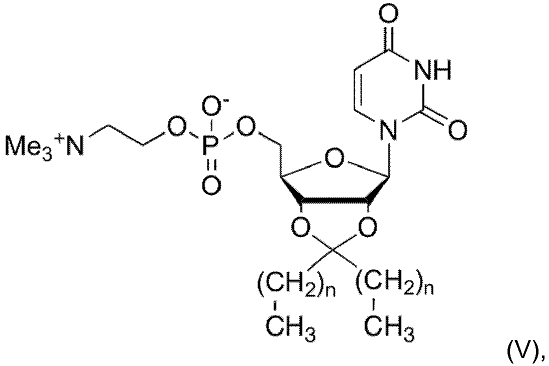 ou
- le groupe défini par la formule (VI) ci-dessous, dans lequel n est un nombre entier compris entre 0 et 19:

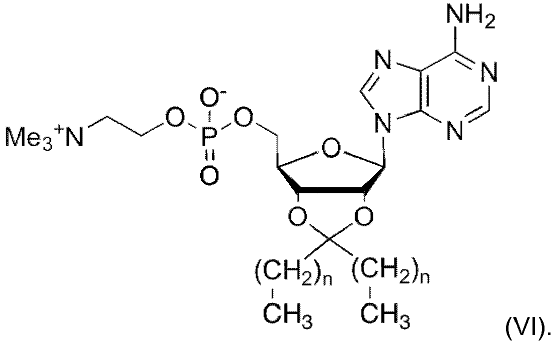

De préférence, l'au moins une molécule à faible poids moléculaire peut être choisie dans le groupe comprenant le:
- 5'-(4-((2*H*,2*H*,3*H*,3*H*-perfluoroundecanamide)methyl)-1*H*-1,2,3-triazole-1-yl)-N3-(1-((β-D-glucopyranoside)-1H-1,2,3-triazole-4-yl)methyl)thymidine,
- 5'-(4-((1H,1H,2H,2H-perfluoroundecanamide)methyl)-1H-1,2,3-triazol-1-yl)-N3-(1-((β-D-glucopyranoside)-1H-1 ,2,3-triazol-4-yl)methyl)thymidine,
- 5'-(4-((Oleamide)methyl)-1H-1,2,3-triazol-1-yl)-N3-(1-((β-D-glucopyranoside)-1H-1,2,3-triazol-4-yl)methyl)thymidine,
- 5'-(4-((stearamide)methyl)-1H-1,2,3-triazol-1-yl)-N3-(1-((β-D-glucopyranoside)-1H-1,2,3-triazol-4-yl)methyl)thymidine
- 5'-(4-((octadecyloxy)methyl)-1H-1,2,3-triazol-1-yl)-N3-(1-((β-D-glucopyranoside)-1H-1,2,3-triazol-4-yl)methyl)thymidine, et
- 5'-(4-((cholesteryloxy)methyl)-1H-1,2,3-triazol-1-yl)-N3-(1-((β-D-glucopyranoside)-1H-1,2,3-triazol-4-yl)methyl)thymidine,
- 2',3'-O-18-pentatriacontanyliden-uridine-5'-phosphocholine,
- 2',3'-O-18-pentatriacontanyliden-adenosine-5'-phosphocholine, et
- un mélange d'au moins deux de ces composés.

De préférence encore, l'au moins une molécule à faible poids moléculaire peut être choisie dans le groupe comprenant:
- le 5'-(4-((2*H*,2*H*,3*H*,3*H*-perfluoroundecanamide)methyl)-1*H*-1,2,3-triazole-1-yl)-N3-(1-((β-D-glucopyranoside)-1H-1,2,3-triazole-4-yl)methyl)thymidine.

Des procédés de fabrication de telles molécules à faible poids moléculaire sont décrits par exemple dans le document WO 2005/116043 **[1].**

Le système supramoléculaire selon l'invention peut comprendre un polymère.

Dans la présente, on entend par « polymère », toute molécule de masse moléculaire élevée constituée de monomère unis les uns aux autres par des liaisons covalentes.

Dans la présente, le polymère est choisi dans legroupe comprenant du collagène, un polysaccharide, un protéoglycane, un glycosaminoglycane (GAG) et le mélange d'au moins deux de ces polymères.

Par exemple, le polymère peut être un polymère synthétique choisi dans le groupe comprenant les polypeptides, les polysaccharides et polynucléotides.

Lorsque le polymère est du collagène, il peut être choisi dans le groupe comprenant le collagène de type I, le collagène de type II et le collagène partiellement hydrolysé. Par exemple, le collagène partiellement hydrolysé peut être de la gélatine.

Lorsque le polymère est un polysaccharide, il peut être choisi dans le groupe comprenant l'amylopectine, l'amylose, la dextrine, les cyclodextrines, l'agar-agar, la carraghénane, la chitine, les xylanes, l'inuline, les dérivés du chitosan, de l'amidon, de la cellulose.

Lorsque le polymère est un protéoglycane, il peut être choisi dans le groupe comprenant le vercican, le perlecan, le neurocan, l'aggrecan et la fibroglycane.

De préférence, le polymère est un glycosaminoglycane (GAG). Un glycosaminoglycane est une chaîne polysaccharidique linéaire, c'est-à-dire non ramifiée, consistant en une répétition d'unité disaccharidique. L'unité disaccharidique consiste en un hexose ou un acide hexuronique, lié à un hexosamine. On entend par « hexosamine » un sucre à six atomes de carbone comprenant un groupement amine.

Lorsque le polymère est un glycosaminoglycane (GAG; ou un mucopolysaccharide (MPS)), il peut être choisi dans le groupe comprenant l'héparine, le hyaluronate de sodium, le kératane sulfate, le sulfate d'héparane, le sulfate de chondroïtine et, le sulfate de dermatane.

Selon l'invention, le polymère peut être un polymère naturel ou synthétique.

Par exemple, le polymère naturel peut être un polymère provenant d'un organisme invertébré ou vertébré.

Par exemple, le polymère naturel peut être un polymère naturel provenant d'un organisme invertébré choisi dans le groupe comprenant la gelée de méduse (« JellyFish » en anglais), de la gangue de Rotifères planctoniques (« Jelly Plankton » en anglais). Par exemple la gelée de méduse est de la gelée de *Cténophore Mnemiopsis.* Par exemple la gangue de Rotifères planctoniques est de la gangue d*'Asplanchna.*

La gelée de méduse et la gangue de Rotifères planctoniques sont constituées de Glycosaminoglycanes (GAGs) et les mucopolysaccharides (MPS).

La mésoglée est capable de piéger les nanoparticules après la mort de la méduse. La captation des nanoparticules ne peut être effective que lorsque la mésoglée (gel) est mise en contact avec le milieu aqueux contaminé.

Selon l'invention, la concentration en système supramoléculaire utilisé, c'est-à-dire la concentration utilisée en molécule à faible poids moléculaire et/ou en polymère définis ci-dessus, est comprise entre 0,001 mg.mL⁻¹ et 100 mg.mL⁻¹ de milieu aqueux. Par exemple cette concentration peut être comprise entre 0,005 mg.mL⁻¹ et 50 mg.mL⁻¹, par exemple entre 0,01 mg.mL⁻¹ et 20 mg.mL⁻¹, par exemple 10 mg.mL⁻¹.

La présente invention a également pour objet un groupe de composés de formule (IV) ci-dessous: 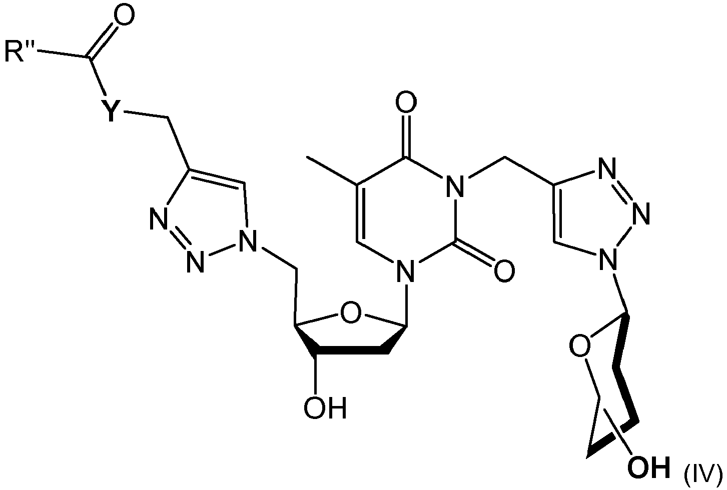 dans laquelle Y est un atome d'oxygène, et R" est une chaîne hydrocarbonée ou une chaîne fluorocarbonnée.

La chaîne hydrocarbonée et la chaîne fluorocarbonnée sont telles que définies ci-dessus.

Des procédés de fabrication de telles molécules à faible poids moléculaire sont décrits par exemple dans le document WO 2005/116043 **[1]**

D'autres avantages pourront encore apparaître à l'homme du métier à la lecture des exemples ci-dessous, illustrés par les figures annexées, donnés à titre illustratif et non limitatif.

### Brève description des figures

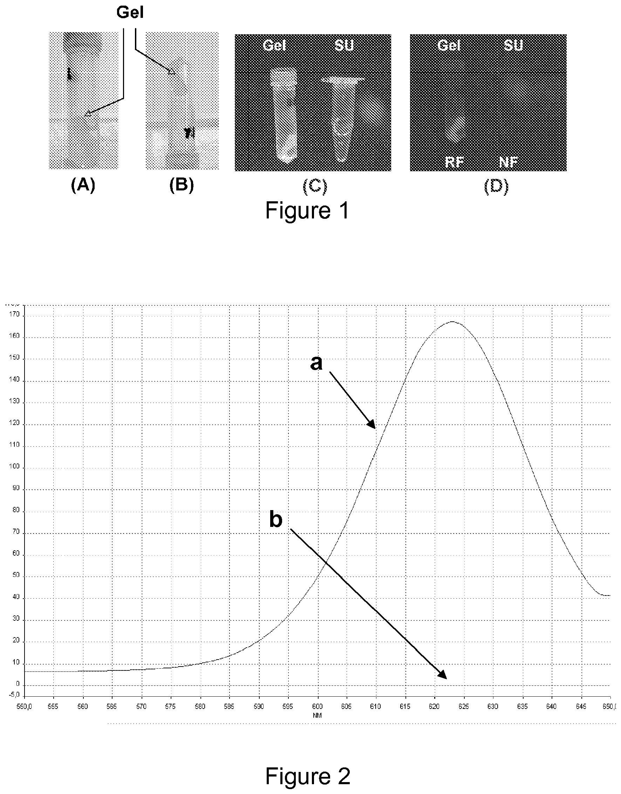

- La figure 1 représente un microtube comprenant un hydrogel et un surnageant d'une solution de QDs encapsulés. En 1A, est représenté le microtube en position normale. En 1B, est représenté le même microtube que 1A en position renversée. E 1C, est représenté l'hydrogel et le surnageant sans UV. En 1D, est représenté le même microtube que 1C sous UV à λₘₐₓ = 312 nm. « SU » signifie Surnageant, « RF » signifie qu'une fluorescence rouge est observée et « NF » signifie qu'aucune fluorescence n'est observée.
- La figure 2 représente un spectre de fluorescence d'une solution de QDs encapsulés dans de l'eau (a) et du surnageant après la formation de l'hydrogel selon le protocole décrit dans cet exemple (b). En abscisse est représentée la longueur d'onde en nanomètre (NM) et en ordonnées est représentée l'intensité en unité arbitraire (I).

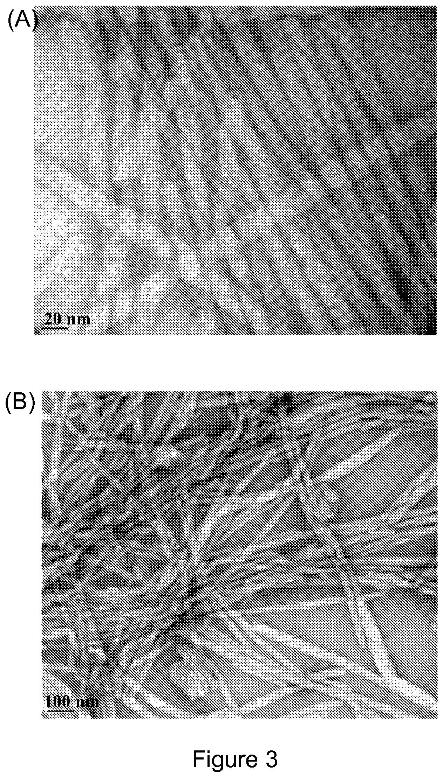

   La figure 3 représente des photos prises au microscope de GNF sans (3A) ou avec (3B) des QDs encapsulés.

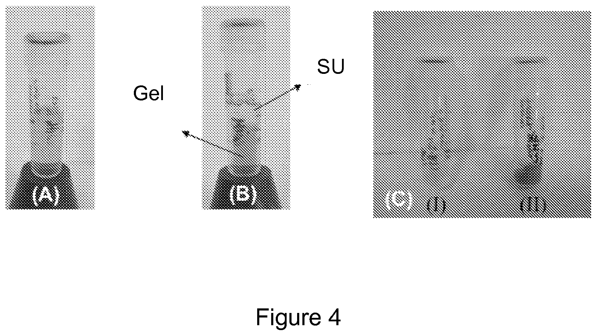

- La figure 4 représente un microtube (a) d'une solution de AuNPs couvertes de L-Lysine dans de l'eau, (b) des AuNPs couvertes de L-Lysine 48 heures après la formation de l'hydrogel de GNFs et (c) du surnageant liquide incolore (I) et du gel de GNFs et d'AuNPs couvertes de L-Lysine de couleur rouge rubis (II).

### EXEMPLES

Le but de des expériences présentées ci-dessous est de démontrer que le système supramoléculaire utilisé selon l'invention permet de décontaminer un milieu liquide contaminé avec des particules.

### Exemple 1: Décontamination d'un milieu liquide contenant des QDs

Dans cet exemple, des GNFs (glycosyl-nucléosidiques amphiphiles fluorés) ont été utilisés en tant que système supramoléculaire comprend des molécules à faible poids moléculaire pour décontaminer un milieu liquide comprenant des Quantum dots (« QDs ») encapsulés.

### Matériel

### Glycosyl-nucléosidiques amphiphiles fluorés (GNFs):

Du 5'-(4-))2*H*,2*H*,3*H*,3*H*-perfluoroundecanamide)methyl)-1*H*-1,2,3-triazole-1-yl)*N*3-)1-((*b*-D-glucopyranoside)-1*H*-1,2,3-triazole-4-yl)methyl)thymidine a été synthétisé selon le protocole décrit dans le document Godeau *et al.* (Godeau et al., Tetrahedon Lett., 2010, vol. 51, p. 1012-1015 **[2]**).

### Quantum Dots (QDs):

Les QDs ont été obtenues auprès de la société Evident Technologies (référence catalogue: ED-C11-TOL-0620). Ces QDs sont à une concentration de 10 mg.mL⁻¹ dans du toluène (24,4 nM). Leur coeur est composé de CdSe et leur coque en ZnS. Ces QDs sont encapsulés dans de l'oxyde de trioctylphosphine (TOPO).

Les « QDs », ont les propriétés optiques uniques. En effet, des QDs de même matière, mais ayant des tailles différentes, peuvent émettre de la lumière de différentes couleurs. La raison physique est l'effet de confinement quantique.

### DOPC:

Le (2 - {[(2S) -2,3-bis [(9Z)-octadéc-9-enoyloxy] propyl phosphonato] oxy} éthyle) trimethylazanium) a été obtenu auprès de la société Genzyme Pharmaceuticals (référence catalogue: LP-R4-070).

### DOTAU:

Le (N-[5'-(2 ', 3'-dioléoyl) uridine]-N', N', N'-triméthylammonium tosylate) a été synthétisé selon le protocole décrit dans le document Chabaud et al. (Chabaud et al., Bioconjugate Chem., 2006, vol. 17, p. 466-472 **[3]**).

### Procédé d'encapsulation des QDs dans des micelles DOTAU/DOPC

200 µl de QDs encapsulés dans du TOPO (10 mg.mL⁻¹ dans du toluène) et 100 mL de chloroforme ont été versés dans une flasque RB. Les solvants ont été évaporés au rotavapeur à 30°C puis les QDs on été séchés sous vide élevé à l'aide d'une pompe à vide pendant 3 à 4 heures.

Les QDs ont ensuite été suspendus dans 35 mL de chloroforme avec 5 ml de phospholipides (2,5 mL DOTU et 2,5 mL DOPC, à une concentration de 10 mg.mL⁻¹ dans le chloroforme).

400 µL de la solution obtenue a ensuite été distribuée dans 100 tubes à essai (400 µL par tube). Après évaporation complète du chloroforme de chaque tube à essai, les résidus ont été chauffés à 80°C et 1 mL d'eau a été ajouté à 50 de ces tubes à essai afin d'obtenir une suspension claire contenant des micelles DOTU/DOPC. Le contenu de chacun de ces 50 tubes à essai a été transféré dans un autre des 50 autres tubes à essai afin d'obtenir une suspension optiquement claire contenant DOTU / DOPC micelles. Ce transfert a été réalisé afin de ne pas avoir un volume total supérieur à 50 ml.

Les QDs encapsulés de phospholipides ont ensuite été transféré dans des microtubes de 2000 µL. Les éventuels agrégats de QDs encapsulé dans les phospholipides ont été éliminés par centrifugation à 40000 rpm à 20-25°C deux fois 15 minutes.

Pour séparer la solution QDs des agrégats résiduels, le surnageant a été recueilli et transféré dans un Vivaspin (Mw seuil de coupure de 30 kDa).

Le volume total de la solution de QDs a été réduit de 20 à 25 mL par centrifugation à 4000 rpm à 20-25°C pendant 15 minutes. Toute la solution de QDs a ensuite été récoltée dans un flacon et stocké dans le noir à 4°C.

La taille des QDs encapsulés obtenus était inférieure ou égale à 20 nm (mesuré sur Zetasizer par des expériences DLS) (Binil Itty Ipe et al., ChemPhysChem, 2006, 7, 1112-1118 **[4]).**

Leur concentration était de 17 microg,mL⁻¹ dans l'eau (mesurée par spectroscopie de fluorescence. Les spectres de fluorescence ont été enregistrés sur un spectrofluorimètre LS 55 (Perkin Elmer) équipé d'une lampe xénon flash. Le traitement des données a été réalisé avec le programme SigmaPlot 11.

### Partie expérimentale: Formation d'un gel pour décontaminer une solution contenant des QDs

0,1 mg de GNF en solution à 0,1% (soit 1,0 mg.mL⁻¹) a été mélangé avec 1 mL de QDs en solution dans de l'eau à une concentration de 17 µg.mL¹ dans un microtube de 2 mL, à 20-25°C.

Un gel a été préparé en chauffant la solution à 80°C dans un bain marie avec agitation constante jusqu'à l'obtention d'une solution visuellement claire. Le temps d'agitation dans cette expérience était de 3 à 4 minutes. La solution a ensuite été laissée 48 heures dans l'obscurité pour qu'elle puisse se stabiliser.

Après 48 heures un gel a été formé avec un surnageant liquide. La présence du gel et du surnageant a été confirmée en retournant le microtube: le gel est maintenu dans le fond du tube alors que le surnageant s'écoule (voir Figure 1A et 1B).

Le surnageant liquide a été séparé du gel. Puis, une observation aux UV (λₘₐₓ = 312 nm) du surnageant liquide a confirmé que les QDs encapsulés ont tous été capturés par le gel. En effet, une fluorescence rouge a été observée pour le gel, alors qu'aune fluorescence n'a été observée pour le surnageant (Figures 1C et 1D).

De plus, la figure 2 présente le spectre de fluorescence d'une solution de QDs encapsulés dans de l'eau (a) et du surnageant après la formation du gel selon le protocole décrit dans cet exemple (b). La capture des QDs encapsulées par le GNF utilisé a été observée au microscope (Figure 3A et 3B).

### Conclusion

Les GNFs (glycosyl-nucléosidiques amphiphiles fluorés) permettent de former un gel au contact du milieu liquide permettant de décontaminer un milieu liquide contaminé avec des particules ayant une taille inférieure à 20 nm (QDs encapsulés).

### Exemple 2: Décontamination d'un milieu liquide contenant des nanoparticules d'or

Dans cet exemple, des GNFs (glycosyl-nucléosidiques amphiphiles fluorés) ont été utilisés en tant que système supramoléculaire comprend des molécules à faible poids moléculaire pour décontaminer un milieu liquide comprenant des nanoparticules d'or (« AuNPs »).

### Matériel

### Glycosyl-nucléosidiques amphiphiles fluorés (GNFs):

Du 5'-(4-))2*H*,2*H*,3*H*,3*H*-perfluoroundecanamide)methyl)-1*H*-1,2,3-triazole-1-yl)*N*3-)1-((*b*-D-glucopyranoside)-1*H*-1,2,3-triazole-4-yl)methyl)thymidine a été synthétisé selon le protocole décrit dans le document Godeau *et al.* (Godeau et al., Tetrahedon Lett., 2010, vol. 51, p. 1012-1015 **[2]**).

### Acide chloroaurique (« HAuCl₄ »):

L'acide chloroaurique a été obtenu auprès de la société Alfa Aesar (Ref N° 36400).

### Procédé de préparation de nanoparticules d'or couvertes par de la L-Lysine

Ce procédé a été réalisé selon le protocole décrit dans Selvakannan et al. (Selvakannan et al., Langmuir, 2003, vol. 19, p. 3545-3549 **[5].**

100 mL de solution aqueuse d'acide chloroaurique (HAuCl₄) à une concentration de 10⁻⁴ M a été réduit par ajout de 0,01 g de borohydrure de sodium (NaBH4) à 20-25°C pour produire des particules d'or colloïdales. Cette procédure aboutit à changement de couleur de la solution du jaune pâle au rouge rubis. Cela indique la formation de nanoparticules d'or.

Les particules d'or colloïdales ont été couvertes par addition de 10 ml d'une solution aqueuse de lysine à 10⁻³ M dans 90 ml de solution de particules d'or colloïdales. Garder de côté pour la nuit. Cela donnera à la L-lysine des nanoparticules d'or plafonné soluble dans l'eau. La solution obtenue a été laissée reposée pendant la nuit, afin de former les AuNPs couvertes de L-Lysine.

Le diamètre moyen des AuNPs non couverte de L-Lysine était de 6,5 nm ± 0,7 nm.

La concentration des AuNPs couvertes de L-Lysine était 10⁻⁴ M.

### Partie expérimentale: Formation d'un gel pour décontaminer une solution contenant des AuNPs couvertes de L-Lysine

0,1 mg de GNF en solution à 0,1% (soit 1,0 mg.mL⁻¹) a été mélangé avec 2 mL de AuNPs couvertes de L-Lysine en solution dans de l'eau à une concentration de 10⁻⁴ M dans un microtube de 2 mL, à 20-25°C.

Un gel a été préparé en chauffant la solution à 80°C dans un bain marie avec agitation constante jusqu'à l'obtention d'une solution visuellement claire. Le temps d'agitation dans cette expérience était de 3-4 minutes. La solution a ensuite été laissée 48 heures dans l'obscurité pour qu'elle puisse se stabiliser.

Après 48 heures un gel a été formé avec un surnageant liquide. La présence du gel et du surnageant a été confirmée en retournant le microtube: le gel est maintenu dans le fond du tube alors que le surnageant s'écoule de la même manière que dans l'exemple 1.

Le surnageant liquide a été séparé du gel. Une couleur rouge rubis a été observée dans le gel alors que le surnageant été incolore, ce qui confirme que toutes les AuNPs couvertes de L-Lysine ont été capturés dans le gel de GNFs.

De plus, la Figure 4 présente le spectre de fluorescence (a) d'une solution de AuNPs couvertes de L-Lysine dans de l'eau, (b) des AuNPs couvertes de L-Lysine 48 heures après la formation du gel de GNFs et (c) du surnageant liquide incolore (I) et du gel de GNFs et d'AuNPs couvertes de L-Lysine de couleur rouge rubis.

### Conclusion

Les GNFs (glycosyl-nucléosidiques amphiphiles fluorés) permettent de former un gel au contact du milieu liquide permettant de décontaminer un milieu liquide contaminé avec des particules d'or couvertes de L-Lysine ayant une taille inférieure à d'environ 6,5 nm.

### Exemple 3: Décontamination d'un milieu liquide contenant des QDs

Dans cet exemple, des polymères naturels ont été utilisés en tant que système supramoléculaire comprend un polymère pour décontaminer un milieu liquide comprenant des Quantum dots (« QDs ») encapsulés.

### Matériel

### Méduses:

Des méduses Cténaires *Mnemiopsis leidyi* (Agassiz, 1865) d'environ 0,5 g grammes et 1 centimètre de diamètre ont été récoltées dans l'étang de Berre (France).

Ces méduses sont constituées de glycosaminoglycans (GAGs) et/ou des mucopolysaccharides.

### Cnidaires Scyphozoaires:

Des Cnidaires Scyphozoaires, Aurelia, de 200 g et 10 centimètres de diamètre ont été récoltés étang de Berre (France).

### Quantum Dots (QDs) encapsulées (DOPC/DOTU):

Les QDs encapsulés ont été obtenus de la même manière que dans l'exemple 1 ci-dessus.

La taille des QDs encapsulés obtenus était inférieure ou égale à 20 nm (mesuré sur Zetasizer par des expériences DLS) (Binil Itty Ipe *et al.* **[4]**).

Leur concentration était de 17 microg.mL⁻¹ dans l'eau (mesurée par spectroscopie de fluorescence. Les spectres de fluorescence on été enregistrés sur un spectrofluorimètre LS 55 (Perkin Elmer) équipé d'une lampe xénon flash. Le traitement des données a été réalisé avec le programme SigmaPlot 11.

### Partie expérimentale: Formation d'un gel pour décontaminer une solution contenant des QDs encapsulées

Une dizaine de méduses *Mnemiopsis* ont été mises en présence avec 1 mL de QDs en solution dans de l'eau à une concentration de 17 µg.mL⁻¹ dans un tube en verre de 5 mL, à 20-25°C.

Le mélange a modérément été agité pendant quelques secondes et a été laissé reposée pendant 2 à 3 jours.

Le tube en verre contenant mélange réactionnel ci-dessus a été examiné sous lumière UV immédiatement après le mélange. On a observé que sous UV le mélange réactionnel entier était rouge fluorescentes, à l'exception des portions de méduses. La portion de méduses apparaissait comme des taches noires et aucune fluorescence n'a été observée. Cela signifie que les QDs encapsulées ont été absorbés uniquement sur la surface de la méduse et ne sont pas capables de pénétrer dans les méduses.

Après 24 heures, les mêmes observations que ci-dessus ont été observées. Les méduses étaient encore vivantes.

Après 48 heures, toutes les méduses étaient mortes et divisés en petits morceaux moelleux. Toute la solution était rouge fluorescente sous UV.

Après 72 heures, toute la solution est devenue transparente avec certains morceaux moelleux précipités au fond du tube de verre ou collés à la surface du tube. Le surnageant liquide a soigneusement été prélevé et a été observé sous UV. De manière surprenant, aucune fluorescence n'a été observée. La quasi totalité des QDs encapsulés ont été absorbés par les méduses mortes. En filtrant ce mélange réactionnel à travers une bande de papier Whatman sous gravité, un filtrat sans aucun QD est obtenu.

Des expériences sur des Cnidaires Scyphozoaires, *Aurelia aurita* (Linnaeus, 1758) de 200 g et 10 centimètres de diamètre fournissent également les mêmes résultats.

### Listes des références

**[1]** WO 2005/116043
**[2]** Godeau et al., Tetrahedon Lett., 2010, vol. 51, p. 1012-1015
**[3]** Chabaud et al., Bioconjugate Chem., 2006, vol. 17, p. 466-472
**[4]** Binil Itty Ipe et al., ChemPhysChem, 2006, 7, 1112-1118
**[5]** Selvakannan et al., Langmuir, 2003, vol. 19, p. 3545-3549

## Revendications

1. Utilisation d'un système supramoléculaire pour soustraire des nanoparticules d'un milieu liquide aqueux les comprenant, dans laquelle ledit système supramoléculaire comprenant au moins une molécule à faible poids moléculaire de formule (I) ci-dessous: 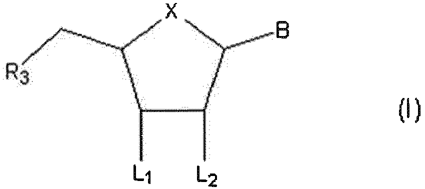 dans laquelle:
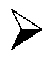 X représente un atome d'oxygène;
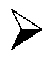 B représente une base purique ou pyrimidique naturelle ou non, éventuellement substituée par un groupement R₃' tel que défini ci-dessous;
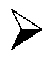 les substituants L₁ et L₂:
ο sont identiques ou différents et représentent:
(i) un atome d'hydrogène,
(ii) un groupe hydroxy,
(iii) un groupe hétéroaryle comprenant 1 à 4 atomes d'azote, non substitué ou substitué par une chaîne hydrocarbonée, saturée ou insaturée, linéaire ou ramifiée en C₂-C₃₀,
(iv) un groupement choisi parmi un groupe oxycarbonyl -O-C(O)-, un groupe thiocarbamate -O-C(S)-NH-, un groupe carbonate -OC(O)-O-, un groupe carbamate -O-C(O)-NH-, un groupement éther -O-, un groupe phosphate et un groupe phosphonate, sachant que ledit groupement L₁ est substitué par un groupement R₁ et ledit groupement L₂ est substitué par un groupement R₂, où R₁ et R₂ identiques ou différents, représentent:
- une chaîne hydrocarbonée, linéaire ou ramifiée, en C₂-C₃₀, de préférence en C₆-C₂₅, plus préférentiellement en C₈-C₂₅, saturée ou insaturée, partiellement fluorée ou perfluorée,
- un radical acyle en C₂-C₃₀, ou
- un groupe acylglycérol,
ou
ο forment un groupement cétal de formule (II) ci-dessous: 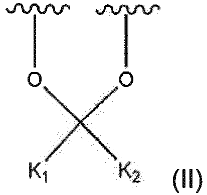
formule (II) dans laquelle K₁ et K₂ sont identiques ou différents et représentent une chaîne hydrocarbonée, saturée ou insaturée, en C₁-C₁₉,
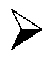 R₃ et R₃'
• représentent indépendamment l'un de l'autre:
(i) un groupement hydroxy, amino, phosphate, phosphonate, phosphocholine, O-alkyl phosphocholine, thiophosphate, phosphonium,
(ii) une chaîne alkyle linéaire ou ramifiée en C₂-C₃₀, éventuellement substituée par au moins un groupe hydroxy,
(iii) un groupe -(CH₂)ₙ-V-R₈, dans lequel V représente un groupe -O-, -S-, ou -NH-, R₈ représente un alkyle en C₂-C₃₀, et n est un entier de 1 à 50,
(iv) un groupe -V-C(O)-R₈, dans lequel V représente un groupe - O-, -S-, ou -NH-, R₈ représente un alkyle en C₂-C₃₀,
ou
(vi) un groupe hétéroaryle renfermant de 1 à 4 atomes d'azote, ledit groupe hétéroaryle étant non substitué ou substitué par un alkyle en C₂-C₃₀, par un groupe (CH₂)ₘ-O-(CH₂)ₚ-R₉, ou un groupe -(CH₂)₀₋₁-Y-C(=O)-R", ou un mono- ou polysaccharide, ou un groupe : 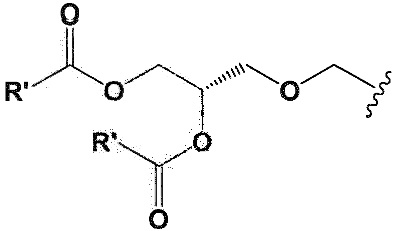 ou par un groupe : 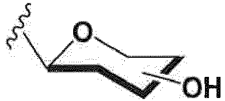 dans lesquels:
m est un entier de 1 à 6,
p est un entier de 0 à 10 et
R₉ représente un groupement alkyl en C₁ à C₁₀, ou un groupe cétal cyclique renfermant 5 à 7 atomes de carbone, ledit groupe cétal cyclique étant non substitué ou
substitué par au moins un alkyle linéaire ou ramifié en C₂-C₃₀, un groupement stérol, un diacyl glycérol, une chaîne hydrofluorocarbonée ou au moins un mono- ou
polysaccharide,
Y est un atome d'oxygène, un groupe NH ou un atome de soufre,
et R" est une chaîne hydrocarbonée ou une chaîne fluorocarbonée, R' est une chaîne hydrocarbonée, ou
• sont liés indépendamment l'un de l'autre par liaison covalente à un autre substituant R₃ ou R₃', identique ou différent, d'un autre composé de formule (I), identique ou différent, pour former un composé sous forme de dimère

2. Utilisation selon la revendication 1, dans laquelle ladite molécule à faible poids moléculaire est un glycosyl-nucléoside fluoré amphiphile.

3. Utilisation selon la revendication 1, dans laquelle
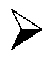 B représente une base thymine, adénine, guanine, cytosine, 6-méthoxypurine, hypoxanthine ou une base purique ou pyrimidique non naturelle qui peut englober la base : 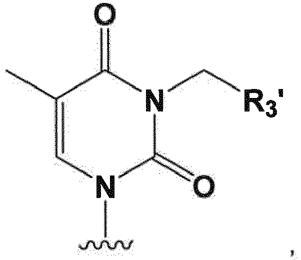 dans laquelle :
R₃' est tel que défini ci-dessous et/ou
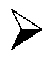 L₁ représente un groupe hydroxy, L₂ représente un atome d'hydrogène, ou L₁ et L₂ forment ensemble un groupement cétal de formule (II) ci-dessous: 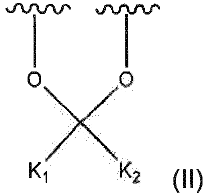 dans laquelle K₁ et K₂ sont identiques ou différents et représentent une chaîne hydrocarbonée, saturée ou insaturée, en C₁-C₁₉ et/ou
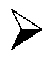 R₃ et R₃'
• représentent:
(i) un groupement hydroxy, amino, phosphate, phosphonate, phosphocholine, O-alkyl phosphocholine, thiophosphate ou phosphonium,
(ii) une chaîne alkyle linéaire ou ramifiée en C₂-C₃₀, éventuellement substituée par au moins un groupe hydroxy,
(iii) un groupe -(CH₂)ₙ-V-R₈, dans lequel V représente un groupe -O-, -S-, ou -NH-, R₈ représente un alkyle en C₂-C₃₀, et n est un entier 1 à 50,
(iv) un groupe -V-C(O)-R₈, dans lequel V représente un groupe - O-, -S-, ou -NH-, R₈ représente un alkyle en C₂-C₃₀,
ou
(v) un groupe hétéroaryle renfermant de 1 à 4 atomes d'azote, ledit groupe hétéroaryle étant non substitué ou substitué par un alkyle en C₂-C₃₀, ou par un groupe (CH₂)ₘ-O-(CH₂)ₚ-R₉, ou un groupe -(CH₂)₀₋₁-Y-C(=O)-R", ou un mono- ou polysaccharide, ou un groupe : 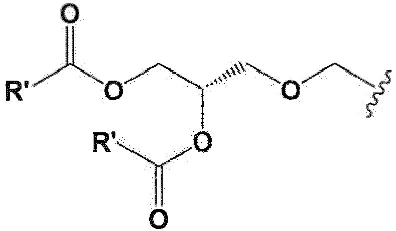 ou un groupe : 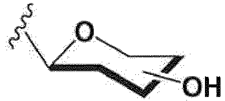 dans lesquels:
m est un entier de 1 à 6,
p est un entier de 0 à 10 et
R₉ représente un groupement alkyl en C₁ à C₁₀, ou un groupe cétal cyclique renfermant 5 à 7 atomes de carbone, ledit groupe cétal cyclique étant non substitué ou substitué par au moins un alkyle linéaire ou ramifié en C₂-C₃₀, un groupement stérol, un diacyl glycérol, une chaîne hydrofluorocarbonée ou au moins un mono- ou polysaccharide,
Y est un atome d'oxygène, un groupe NH, ou un atome de soufre,
et R" est une chaîne hydrocarbonée ou une chaîne fluorocarbonée,
R' est une chaîne hydrocarbonée, ou
• sont liés indépendamment l'un de l'autre par liaison covalente à un autre substituant R₃ ou R₃', identique ou différent, d'un autre composé de formule (I), identique ou différent, pour former un composé sous forme de dimère.

4. Utilisation selon la revendication 1, dans laquelle
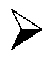 B représente une base pyrimidique non naturelle substituée par un groupe hétéroaryle renfermant 3 atomes d'azote, ledit groupe hétéroaryle étant substitué par un groupe: 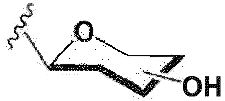
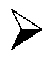 les substituants L₁ et L₂ sont identiques ou différents et représentent:
(i) un atome d'hydrogène,
(ii) un groupe hydroxy,
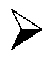 R₃
• représente un groupe hétéroaryle renfermant 3 atomes d'azote, ledit groupe hétéroaryle étant substitué par un groupe : 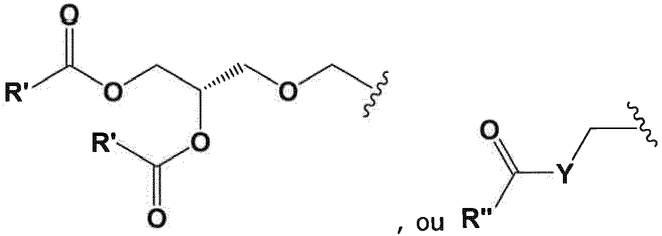 dans lesquels :
R' est une chaîne hydrocarbonée;
Y est un atome d'oxygène, un groupe NH, ou un atome de soufre,
et R" est une chaîne hydrocarbonée ou une chaîne fluorocarbonnée.
ou
• est lié à un autre substituant R₃, identique ou différent, d'un autre composé de formule (I), identique ou différent, pour former un composé sous forme de dimère.

5. Utilisation selon la revendication 1, dans laquelle
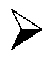 B représente une base purique ou pyrimidique;
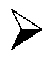 les substituants L₁ et L₂ forment un groupement cétal de formule (II) ci-dessous: 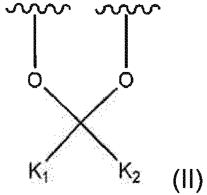 formule (II) dans laquelle K₁ et K₂ sont identiques ou différents et représentent une chaîne hydrocarbonée, saturée ou insaturée, en C₁-C₁₉,
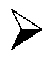 R₃
• représente un groupement phosphocholine ; ou
• est lié à un autre substituant R₃, identique ou différent, d'un autre composé de formule (I), identique ou différent, pour former un composé sous forme de dimère.

6. Utilisation selon la revendication 1, dans laquelle ladite molécule à faible poids moléculaire est choisie dans:
- le groupe défini par la formule (III) ci-dessous, dans lequel R' est une chaîne hydrocarbonée: 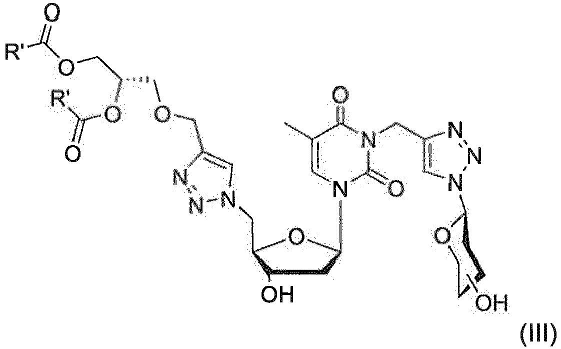
- le groupe défini par la formule (IV) ci-dessous, dans lequel Y est un atome d'oxygène, un groupe NH ou un atome de soufre, et R" est une chaîne hydrocarbonée ou une chaîne fluorocarbonée: 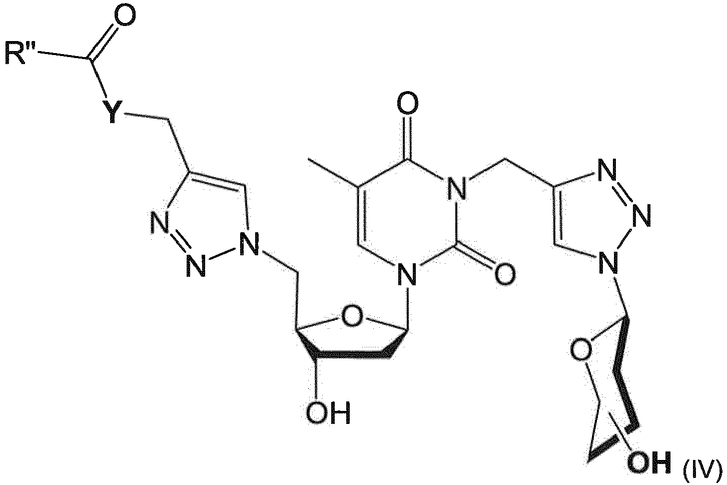
- le groupe défini par la formule (V) ci-dessous, dans lequel n est un nombre entier compris entre 0 et 19: 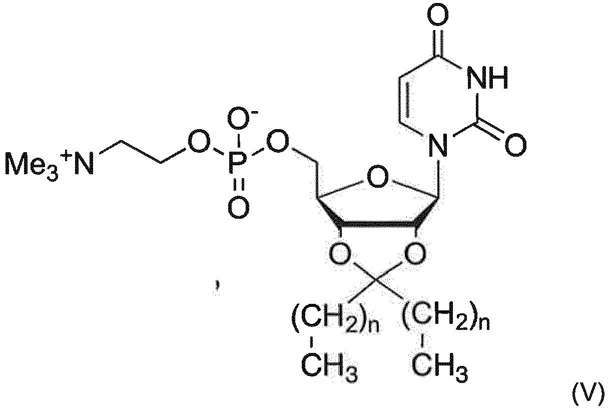 ou
- le groupe défini par la formule (VI) ci-dessous, dans lequel n est un nombre entier compris entre 0 et 19:

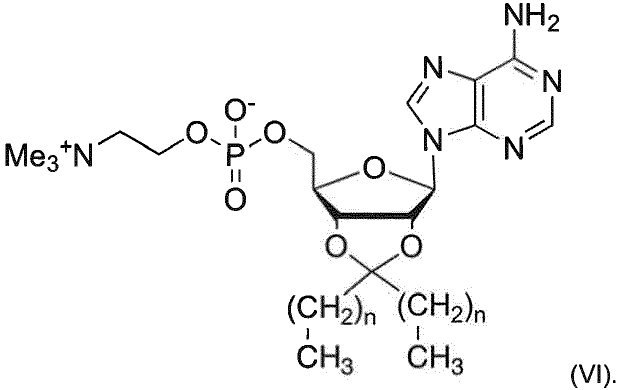

7. Utilisation selon la revendication 1, dans laquelle ladite molécule à faible poids moléculaire est choisie dans le groupe comprenant:
- 5'-(4-((2*H*,2*H*,3*H*,3*H*-perfluoroundecanamide)methyt)-1*H*-1,2,3-triazole-1-yl)-N3-(1-((β-D-glucopyranoside)-1H-1,2,3-triazole-4-yl)methyl)thymidine,
- 5'-(4-((1*H*,1H,2H,2H-perfluoroundecanamide)methyl)-1H-1,2,3-triazol-1-yl)-N3-(1-((β-D-glucopyranoside)-1H-1 ,2,3-triazol-4-yl)methyl)thymidine,
- 5'-(4-((Oleamide)methyl)-1H-1,2,3-triazol-1-yl)-N3-(1-((β-D-glucopyranoside)-1H-1,2,3-triazol-4-yl)methyl)thymidine,
- 5'-(4-((stearamide)methyl)-1H-1,2,3-triazol-1-yl)-N3-(1-((β-D-glucopyranoside)-1H-1,2,3-triazol-4-yl)methyl)thymidine
- 5'-(4-((octadecyloxy)methyl)-1H-1,2,3-triazol-1-yl)-N3-(1-((β-D-glucopyranoside)-1H-1,2,3-triazol-4-yl)methyl)thymidine, et
- 5'-(4-((cholesteryloxy)methyl)-1H-1,2,3-triazol-1-yl)-N3-(1-((β-D-glucopyranoside)-1H-1,2,3-triazol-4-yl)methyl)thymidine,
- 2',3'-O-18-pentatriacontanyliden-uridine-5'-phosphocholine,
- 2',3'-O-18-pentatriacontanyliden-adenosine-5'-phosphocholine, et un mélange d'au moins deux de ces composés

8. Utilisation selon l'une quelconque des revendications 1 à 7, dans laquelle la concentration en système supramoléculaire utilisé est comprise entre 0,001 mg.mL⁻¹ et 100 mg.mL⁻¹ de milieu aqueux.

9. Utilisation selon l'une quelconque des revendications 1 à 8, dans laquelle les nanoparticules ont une taille comprise entre 0,5 nm et 5 µm, avantageusement entre 5 et 100 nm.

10. Utilisation selon l'une quelconque des revendications 1 à 9, dans laquelle le milieu liquide aqueux est de l'eau.

11. Procédé de soustraction de nanoparticules d'un milieu liquide aqueux les contenant comprenant une étape (a) d'ajout d'un système supramoléculaire dans ledit milieu liquide aqueux à une température comprise entre 2 et 95 °C, dans lequel le système supramoléculaire comprend au moins une molécule à faible poids moléculaire de formule (I) telle que définie dans l'une quelconque des revendications 1 à 7, ledit système supramoléculaire formant un gel au contact du milieu liquide aqueux, ledit gel captant les nanoparticules contenues dans ledit milieu liquide aqueux, et une étape (b) de séparation dudit milieu liquide aqueux et dudit gel ayant capté lesdites nanoparticules.

12. Procédé selon la revendication 11, comprenant en outre avant l'étape (b), lorsque la température du milieu liquide aqueux obtenu à l'étape (a) est inférieure à 50 °C, les étapes intermédiaires suivantes:
(a1) chauffage du milieu obtenu à l'étape (a) à une température comprise entre 50 et 95°C, et
(a2) refroidissement du milieu obtenu à l'étape (a1) à une température comprise entre 2 et 50°C.

13. Procédé selon la revendication 11 ou 12, dans lequel la concentration en système supramoléculaire utilisé est comprise entre 0,001 mg.mL⁻¹ et 100 mg.mL⁻¹ de milieu aqueux.

14. Procédé selon l'une quelconque des revendications 11 à 13, dans lequel les particules ont une taille comprise entre 0,5 nm et 5 µm, avantageusement entre 5 nm et 100 nm.

15. Procédé selon l'une quelconque des revendications 11 à 14, dans lequel le milieu liquide aqueux est de l'eau.

16. Groupe de composés de formule (IV) ci-dessous : 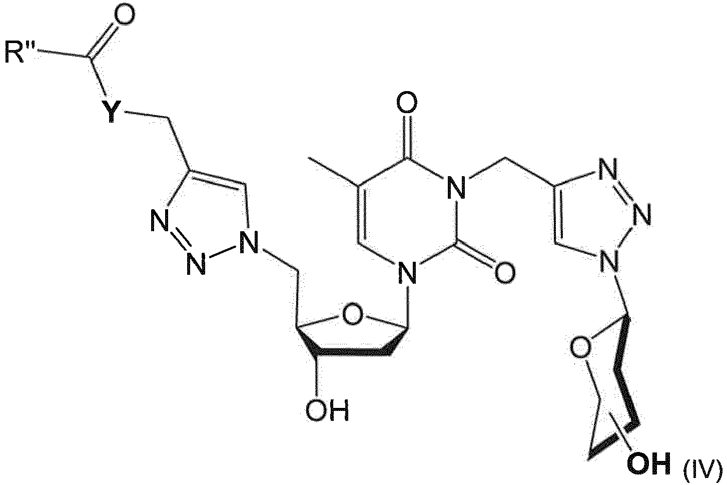 dans lequel Y est un atome d'oxygène, et R" est une chaîne hydrocarbonée ou une chaîne fluorocarbonée.

## Patentansprüche

1. Verwendung eines supramolekulären Systems zum Subtrahieren von Nanopartikel von einem wässrigen flüssigen Medium, das sie umfasst, wobei das supramolekuläre System mindestens ein Molekül mit geringem Molekulargewicht mit der unten stehenden Formel (I) umfasst: 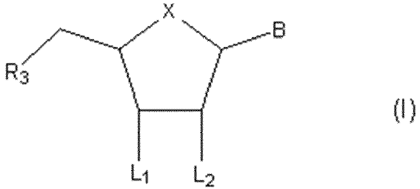 wobei:
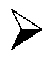 X ein Sauerstoffatom darstellt;
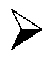 B eine Purin- oder Pyrimidinbase, natürlich oder nicht, darstellt, eventuell substituiert durch ein R₃'-Gruppierung, wie unten definiert;
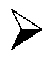 die Substituenten L₁ und L₂:
∘ identisch oder verschieden sind und Folgendes darstellen:
(i) ein Wasserstoffatom,
(ii) eine Hydroxygruppe,
(iii) eine Heteroarylgruppe, umfassend 1 bis 4 Stickstoffatome, nicht substituiert oder substituiert durch eine Kohlenwasserstoffkette, gesättigt oder nicht gesättigt, linear oder verzweigt, mit C₂-C₃₀,
(iv) eine Gruppierung, ausgewählt aus einer Oxycarbonylgruppe -O-C(O)-, einer Thiocarbamatgruppe - O-C(S)-NH-, einer Carbonatgruppe -O-C(O)-O-, einer Carbamatgruppe -O-C(O)-NH-, einer Ethergruppierung -0-, einer Phosphatgruppe und einer Phosphonatgruppe, im Wissen, dass die Gruppierung L₁ durch eine Gruppierung R₁ ersetzt ist und die Gruppierung L₂ durch eine Gruppierung R₂ ersetzt ist, wobei R₁ und R₂, identisch oder verschieden, Folgendes darstellen:
- eine Kohlenwasserstoffkette, linear oder verzweigt, mit C₂-C₃₀, vorzugsweise mit C₆-C₂₅, noch bevorzugter mit C₈-C₂₅, gesättigt oder nicht gesättigt, teilweise fluoriert oder perfluoriert,
- ein Alcylradikal mit C₂-C₃₀, oder
- eine Acylglycerolgruppe,
oder
∘ eine Ketalgruppierung mit der unten stehenden Formel (II) bilden: 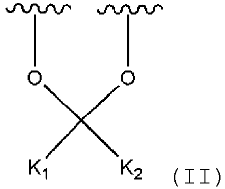 Formel (II), in der K₁ und K₂ identisch oder verschieden sind und eine Kohlenwasserstoffkette darstellen, gesättigt oder nicht gesättigt, mit C₁-C₁₉,
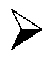 R₃ und R₃'
• unabhängig voneinander Folgendes darstellen:
(i) eine Hydroxy-, Amino-, Phosphat-, Phosphonat-, Phosphocholin-, O-alkylphosphocholin-, Thiophosphat-, Phosphonium-Gruppierung,
(ii) eine Alkylkette, linear oder verzweigt, mit C₂-C₃₀, eventuell substituiert durch mindestens eine Hydroxygruppe,
(iii) eine -(CH₂)ₙ-V-R₈-Gruppe, in der V eine Gruppe -O-, -S-, oder -NH- darstellt, R₈ ein Alkyl mit C₂-C₃₀, darstellt und n eine ganze Zahl von 1 bis 50 ist,
(iv) eine Gruppe -V-C(O)-R₈, in der V eine Gruppe - O-, -S-, oder -NH- darstellt, R₈ ein Alkyl mit C₂-C₃₀ darstellt,
oder
(vi) eine Heteroarylgruppe, die 1 bis 4 Stickstoffatome einschließt, wobei die Heteroarylgruppe nicht substituiert oder substituiert ist durch ein Alkyl mit C₂-C₃₀, durch eine Gruppe (CH₂)ₘ-O-(CH₂)ₚ-R₉ oder eine Gruppe - (CH₂)₀₋₁-Y-C (=O)-R'', oder ein Mono- oder Polysaccharid, oder eine Gruppe: 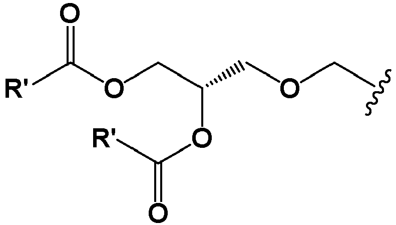 oder durch eine Gruppe: 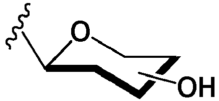 wobei:
m eine ganze Zahl von 1 bis 6 ist,
p eine ganze Zahl von 0 bis 10 ist, und
R₉ eine Alkylgruppierung mit C₁ bis C₁₀ darstellt, oder eine cyclische Ketalgruppe, die 5 bis 7 Kohlenstoffatome einschließt, wobei die cyclische Ketalgruppe nicht substituiert oder substituiert ist durch mindestens ein lineares oder verzweigtes Alkyl mit C₂-C₃₀, eine Sterolgruppierung, ein Diacylglycerol, eine Fluorkohlenwasserstoffkette, und mindestens ein Mono- oder Polysaccharid,
Y ein Sauerstoffatom, eine Gruppe NH oder ein Schwefelatom ist,
und R" eine Kohlenwasserstoffkette oder eine Fluorkohlenstoffkette ist,
R' eine Kohlenwasserstoffkette ist
oder
• unabhängig voneinander durch kovalente Bindung an einen anderen Substituenten R₃ oder R₃', identisch oder verschieden, einer anderen Verbindung mit der Formel (I), identisch oder verschieden, gebunden sind, um eine Verbindung in Form eines Dimers zu bilden.

2. Verwendung nach Anspruch 1, wobei das Molekül mit geringem Molekulargewicht ein amphiphiles fluoriertes Glycosilnucleosid ist.

3. Verwendung nach Anspruch 1, wobei
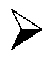 B ein Thymin, Adenin, Guanin, Cytosin, 6-Methoxypurin, Hypoxanthin oder eine Purin- oder nicht natürliche Pyrimidinbase darstellt, die die folgende Base einschließen kann: 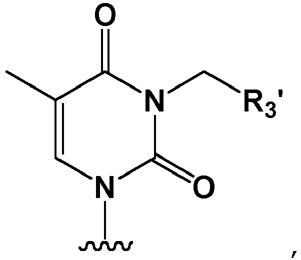 wobei:
R₃' wie unten definiert ist, und/oder
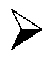 L₁ eine Hydroxygruppe darstellt, L₂ ein Wasserstoffatom darstellt oder L₁ und L₂ zusammen eine Ketalgruppierung mit der unten stehenden Formel (II) bilden: 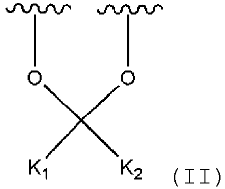 wobei K₁ und K₂ identisch oder verschieden sind und eine Kohlenwasserstoffkette darstellen, gesättigt oder nicht gesättigt, mit C₁-C₁₉ und/oder
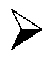 R₃ und R₃'
• Folgendes darstellen:
(i) eine Hydroxy-, Amino-, Phosphat-, Phosphonat-, Phosphocholin-, O-alkylphosphocholin-, Thiophosphat-, oder Phosphonium-Gruppierung,
(ii) eine Alkylkette, linear oder verzweigt, mit C₂-C₃₀, eventuell substituiert durch mindestens eine Hydroxygruppe,
(iii) eine Gruppe -(CH₂)ₙ-V-R₈-, in der V eine Gruppe -O-, -S-, oder -NH- darstellt, R₈ ein Alkyl mit C₂-C₃₀ darstellt und n eine ganze Zahl von 1 bis 50 ist,
(iv) eine Gruppe -V-C(O)-R₈, in der V eine Gruppe - O-, -S-, oder -NH- darstellt, R8 ein Alkyl mit C₂-C₃₀ darstellt, oder
(v) eine Heteroarylgruppe, die 1 bis 4 Stickstoffatome einschließt, wobei die Heteroarylgruppe nicht substituiert oder substituiert ist durch ein Alkyl mit C₂-C₃₀, oder durch eine Gruppe (CH₂)ₘ-O-(CH₂)ₚ-R₉, oder eine Gruppe -(CH₂) ₀₋₁-Y-C(=O)-R", oder ein Mono- oder Polysaccharid, oder eine Gruppe: 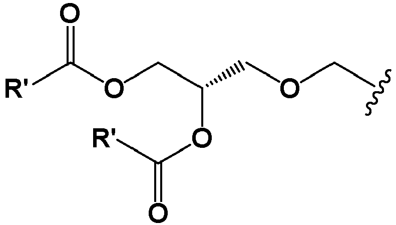 oder eine Gruppe: 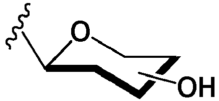 wobei:
m eine ganze Zahl von 1 bis 6 ist,
p eine ganze Zahl von 0 bis 10 ist, und
R₉ eine Alkylgruppierung mit C₁ bis C₁₀ darstellt, oder eine cyclische Ketalgruppe, die 5 bis 7 Kohlenstoffatome einschließt, wobei die cyclische Ketalgruppe nicht substituiert oder substituiert ist durch mindestens ein lineares oder verzweigtes Alkyl mit C₂-C₃₀, eine Sterolgruppierung, ein Diacylglycerol, eine Fluorkohlenwasserstoffkette, und mindestens ein Mono- oder Polysaccharid,
Y ein Sauerstoffatom, ein Gruppe NH oder ein Schwefelatom ist,
und R" eine Kohlenwasserstoffkette oder eine Fluorkohlenstoffkette ist,
R' eine Kohlenwasserstoffkette ist
oder
• unabhängig voneinander durch kovalente Bindung an einen anderen Substituenten R₃ oder R₃', identisch oder verschieden, einer anderen Verbindung mit der Formel (I), identisch oder verschieden, gebunden sind, um eine Verbindung in Form eines Dimers zu bilden.

4. Verwendung nach Anspruch 1, wobei
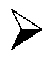 B eine nicht natürliche Pyrimidinbase darstellt, substituiert durch eine Heteroarylgruppe, die 3 Stickstoffatome einschließt, wobei die Heteroarylgruppe substituiert ist durch die folgende Gruppe: 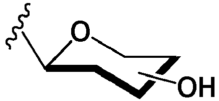
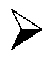 die Substituenten L₁ und L₂, identisch oder verschieden sind und Folgendes darstellen:
(i) ein Wasserstoffatom,
(ii) eine Hydroxygruppe,
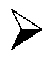 R₃
• eine Heteroarylgruppe darstellt, die 3 Stickstoffatome einschließt, wobei die Heteroarylgruppe substituiert ist durch eine folgende Gruppe: 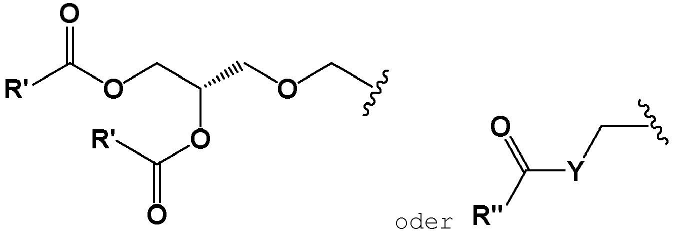 wobei:
R' eine Kohlenwasserstoffkette ist;
Y ein Sauerstoffatom, eine Gruppe NH oder ein Schwefelatom ist,
und R" eine Kohlenwasserstoffkette oder eine Fluorkohlenstoffkette ist,
oder
• an einen anderen Substituenten R₃, identisch oder verschieden, einer anderen Verbindung mit der Formel (I), identisch oder verschieden, gebunden ist, um eine Verbindung in Form eines Dimers zu bilden.

5. Verwendung nach Anspruch 1, wobei
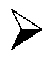 B eine Purin- oder Pyrimidinbase darstellt;
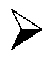 die Substituenten L₁ und L₂ eine Ketalgruppierung mit der unten stehenden Formel (II) bilden: 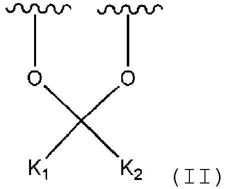 Formel (II), in der K₁ und K₂ identisch oder verschieden sind und eine Kohlenwasserstoffkette darstellen, gesättigt oder nicht gesättigt, mit C₁-C₁₉,
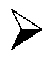 R₃
• eine Phosphocholingruppierung darstellt;
oder
• an einen weiteren Substituenten R₃, identisch oder verschieden, einer weiteren Verbindung mit der Formel (I), identisch oder verschieden, gebunden ist, um eine Verbindung in Form eines Dimers zu bilden.

6. Verwendung nach Anspruch 1, wobei das Molekül mit geringem Molekulargewicht ausgewählt ist aus:
- der Gruppe, definiert durch die unten stehende Formel (III), in der R' eine Kohlenwasserstoffkette ist: 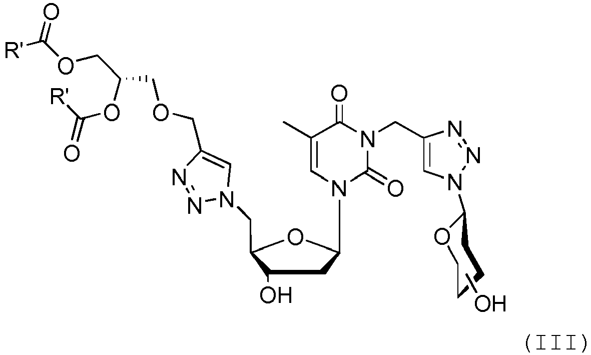
- der Gruppe, definiert durch die unten stehende Formel (IV), in der Y ein Sauerstoffatom, eine Gruppe NH oder ein Schwefelatom ist, und R" eine Kohlenwasserstoffkette oder eine Fluorkohlenstoffkette ist: 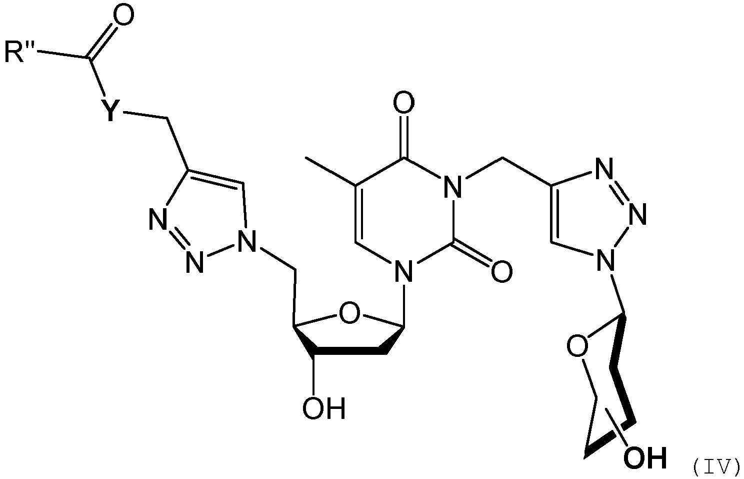
- der Gruppe, definiert durch die unten stehende Formel (V), in der n eine ganze Zahl zwischen 0 und 19 ist: 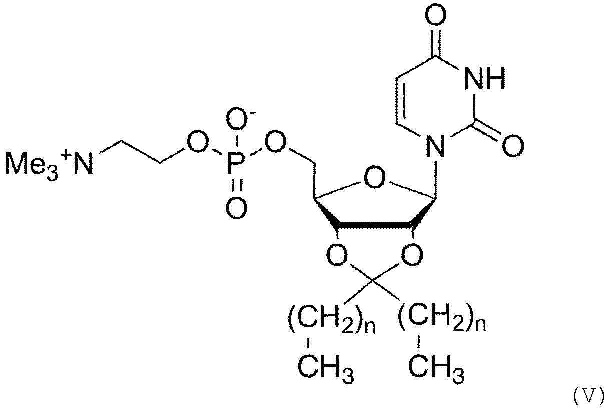 oder
- der Gruppe, definiert durch die unten stehende Formel (VI), in der n eine ganze Zahl zwischen 0 und 19 ist:

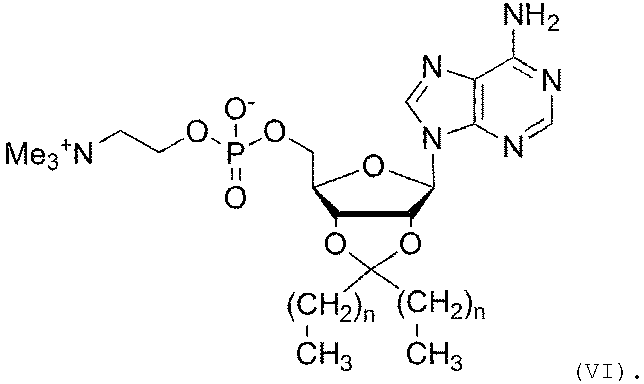

7. Verwendung nach Anspruch 1, wobei das Molekül mit geringem Molekulargewicht ausgewählt ist aus der Gruppe, umfassend:
- 5'-(4-((2*H*,2*H*,3*H*,3*H-*Perfluoroundecanamid)methyl)-1*H*-1,2,3-triazol-1-yl)-N3-(1-((β-D-glucopyranosid)-1H-1,2,3-triazol-4-yl)methyl)thymidin,
- 5"-(4-((1H,1H,2H,2H-Perfluoroundecanamid)methyl)-1H-1,2,3-triazol-1-yl)-N3-(1-((β-D-glucopyranosid)-1H-1,2,3-triazol-4-yl)methyl)thymidin,
- 5"-(4-((Oleamid)methyl)-1H-1,2,3-triazol-1-yl)-N3-(1-((β-D-glucopyranosid)-1H-1,2,3-triazol-4-yl)methyl)thymidin,
- 5'-(4-((Stearamid)methyl)-1H-1,2,3-triazol-1-yl)-N3-(1-((β-D-glucopyranosid)-1H-1,2,3-triazol-4-yl)methyl)thymidin
- 5"-(4-((Octadecyloxy)methyl)-1H-1,2,3-triazol-1-yl)-N3-(1-((β-D-glucopyranosid)-1H-1,2,3-triazol-4-yl)methyl)thymidin, und
- 5"-(4-((Cholesteryloxy)methyl)-1H-1,2,3-triazol-1-yl)-N3-(1-((β-D-glucopyranosid)-1H-1,2,3-triazol-4-yl)methyl)thymidin,
- 2',3'-O-18-Pentatriacontanyliden-uridin-5'-phosphocholin,
- 2',3'-O-18-Pentatriacontanyliden-adenosin-5'-phosphocholin, und
eine Mischung von mindestens zwei dieser Verbindungen.

8. Verwendung nach einem der Ansprüche 1 bis 7, wobei die Konzentration im verwendeten supramolekulären System im Bereich zwischen 0,001 mg.mL⁻¹ und 100 mg.mL⁻¹ wässerigem Medium liegt.

9. Verwendung nach einem der Ansprüche 1 bis 8, wobei die Nanopartikel eine Größe im Bereich zwischen 0,5 nm und 5 nm, vorzugsweise zwischen 5 und 100 nm liegt.

10. Verwendung nach einem der Ansprüche 1 bis 9, wobei das wässrige flüssige Medium Wasser ist.

11. Verfahren zum Subtrahieren von Nanopartikeln von einem wässrigen flüssigen Medium, das sie enthält, umfassend einen Schritt (a) des Zugebens eines supramolekulären Systems in das wässrige flüssige Medium bei einer Temperatur im Bereich zwischen 2 und 95 °C, wobei das supramolekuläre System mindestens ein Molekül mit geringem Molekulargewicht mit der Formel (I) umfasst, wie in einem der Ansprüche 1 bis 7 definiert, wobei das supramolekuläre System beim Kontakt mit dem wässrigen flüssigen Medium ein Gel bildet, wobei das Gel die Nanopartikel einfängt, die im wässrigen flüssigen Medium enthalten sind, und einen Schritt (b) des Trennens des wässrigen flüssigen Mediums und des Gels, dass die Nanopartikel eingefangen hat.

12. Verfahren nach Anspruch 11, umfassend außerdem, vor dem Schritt (b), wenn die Temperatur des wässrigen flüssigen Mediums, erhalten in Schritt (a), geringer als 50 °C ist, die folgenden Zwischenschritte:
(a1) Erhitzen des Mediums, erhalten in Schritt (a), auf eine Temperatur im Bereich zwischen 50 und 95 °C, und
(a2) Abkühlen des Mediums, erhalten in Schritt (a1), auf eine Temperatur im Bereich zwischen 2 und 50 °C.

13. Verfahren nach Anspruch 11 oder 12, wobei die Konzentration an verwendetem supramolekulärem System im Bereich zwischen 0,001 mg.mL⁻¹ und 100 mg.mL⁻¹ wässerigem Medium liegt.

14. Verfahren nach einem der Ansprüche 11 bis 13, wobei die Partikel eine Größe aufweisen, die im Bereich zwischen 0,5 nm und 5 µm, vorzugsweise zwischen 5 nm und 100 nm liegt.

15. Verfahren nach einem der Ansprüche 11 bis 14, wobei das wässrige flüssige Medium Wasser ist.

16. Gruppe von Verbindungen der unten stehenden Formel (IV): 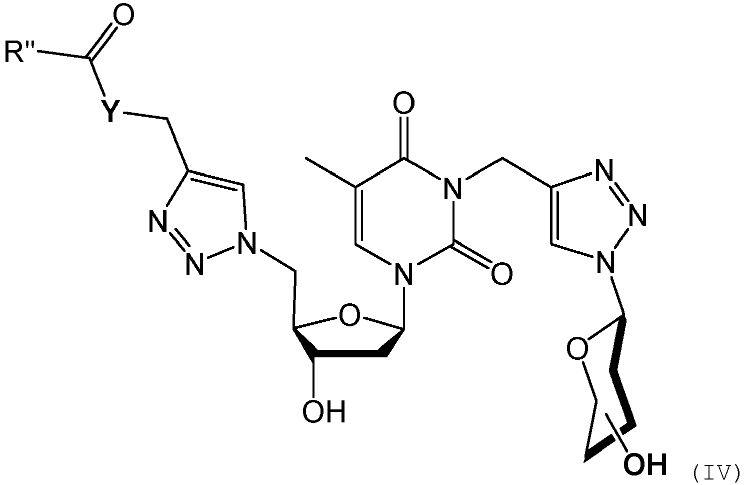 in der Y ein Sauerstoffatom ist und R" eine Kohlenwasserstoffkette oder eine Fluorkohlenstoffkette ist.

## Claims

1. Use of a supramolecular system for subtracting nanoparticles from a liquid medium comprising same, wherein said supramolecular system comprises at least one low-molecular-weight molecule of formula (I) below: 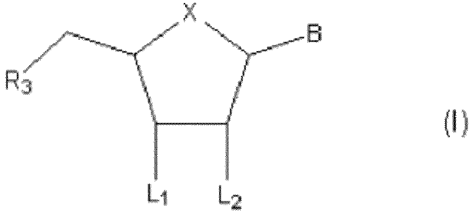 wherein:
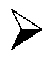 X represents an oxygen atom;
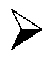 B represents a natural or unnatural puric or pyrimidic base, optionally substituted with an R₃' group as defined below;
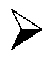 substituents L₁ and L₂:
∘ are identical or different and represent:
(i) a hydrogen atom,
(ii) a hydroxyl group,
(iii) a heteroaryl group comprising 1 to 4 nitrogen atoms, unsubstituted or substituted with a saturated or unsaturated, linear or branched C₂-C₃₀ hydrocarbon-based chain,
(iv) a group chosen from an oxycarbonyl group -O-C(O)-, a thiocarbamate group -O-C(S)-NH-, a carbonate group -O-C(O)-O-, a carbamate group -O-C(O)-NH-, an ether group -0-, a phosphate group or a phosphonate group, knowing that said L₁ group is substituted with an R₁ group and said L₂ group is substituted with an R₂ group, where R₁ and R₂, which may be identical or different, represent:
- a linear or branched, saturated or unsaturated, partially fluorinated or perfluorinated, C₂-C₃₀, preferably C₆-C₂₅, more preferentially C₈-C₂₅, hydrocarbon-based chain,
- a C₂-C₃₀ acyl radical, or
- an acylglycerol group,
or
∘ form a ketal group of formula (II) below: 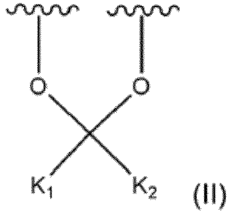
formula (II) in which K₁ and K₂ are identical or different and represent a saturated or unsaturated C₁-C₁₉ hydrocarbon-based chain,
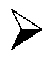 R₃ and R₃'
• represent, independently of one another:
(i) a hydroxyl, amino, phosphate, phosphonate, phosphocholine, O-alkyl phosphocholine, thiophosphate or phosphonium group,
(ii) a linear or branched C₂-C₃₀ alkyl chain optionally substituted with at least one hydroxyl group,
(iii) a -(CH₂)ₙ-V-R₈ group, wherein V represents an -0-, -S- or -NH- group, R₈ represents a C₂-C₃₀ alkyl, and n is an integer from 1 to 50,
(iv) a -V-C(O)-R₈ group, wherein V represents an -O-, -S- or -NH- group, and R₈ represents a C₂-C₃₀ alkyl,
or
(v) a heteroaryl group containing from 1 to 4 nitrogen atoms, said heteroaryl group being unsubstituted or substituted with a C₂-C₃₀ alkyl, or with a (CH₂) ₘ-O- (CH₂) ₚ-R₉ group, or with a - (CH₂) ₀₋₁-Y-C (=O)-R" group, or with a monosaccharide or polysaccharide, or a group: 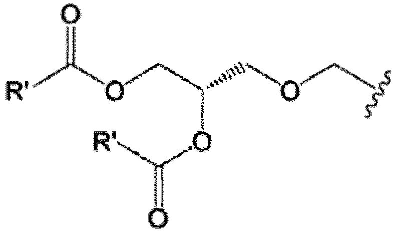 or with a group: 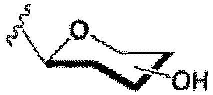 wherein:
m is an integer from 1 to 6,
p is an integer from 0 to 10 and
R₉ represents a C₁ to C₁₀ alkyl group, or a cyclic ketal group containing 5 to 7 carbon atoms, said cyclic ketal group being unsubstituted or substituted with at least one linear or branched C₂-C₃₀ alkyl, a sterol group, a diacyl glycerol, a hydrofluorocarbon-based chain or at least one monosaccharide or polysaccharide,
Y is an oxygen atom, an NH group or a sulfur atom,
and R" is a hydrocarbon-based chain or a fluorocarbon-based chain,
R' is a hydrocarbon-based chain, or
• are linked, independently of one another, via a covalent bond to another substituent R₃ or R₃', identical or different, of another compound of formula (I), identical or different, so as to form a compound in dimer form.

2. The use according to claim 1, wherein said low-molecular-weight molecule is an amphiphilic fluorinated glycosyl nucleoside.

3. The use according to claim 1, wherein
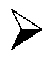 B represents a thymine, adenine, guanine, cytosine, 6-methoxypurine or hypoxanthine or an unnatural puric or pyrimidic base, which can encompass the base: 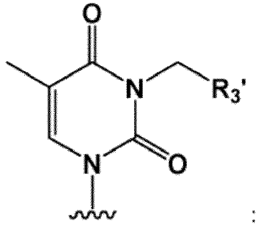 wherein:
R₃' is as defined below,
and/or
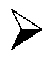 L₁ represents a hydroxyl group, L₂ represents a hydrogen atom, or L₁ and L₂ together form a ketal group of formula (II) below: 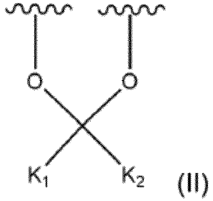 wherein K₁ and K₂ are identical or different and represent a saturated or unsaturated C₁-C₁₉ hydrocarbon-based chain and/or
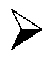 R₃ and R₃'
• represent:
(i) a hydroxyl, amino, phosphate, phosphonate, phosphocholine, O-alkyl phosphocholine, thiophosphate or phosphonium group,
(ii) a linear or branched C₂-C₃₀ alkyl chain, optionally substituted with at least one hydroxyl group,
(iii) a -(CH₂)ₙ-V-R₈ group, wherein V represents an -O-, -S- or -NH- group, R₈ represents a C₂-C₃₀ alkyl, and n is an integer from 1 to 50,
(iv) a -V-C(O)-R₈ group, wherein V represents an -O-, -S- or -NH- group, and R₈ represents a C₂-C₃₀ alkyl, or
(v) a heteroaryl group containing from 1 to 4 nitrogen atoms, said heteroaryl group being unsubstituted or substituted with a C₂-C₃₀ alkyl, or with a (CH₂)ₘ-O-(CH₂)ₚ-R₉ group, or with a - (CH₂) ₀₋₁-Y-C(=O)-R" group, or with a monosaccharide or polysaccharide, or with a group: 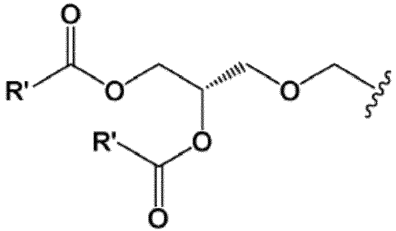 or with a group: 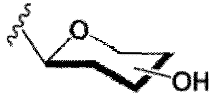 wherein:
m is an integer from 1 to 6,
p is an integer from 0 to 10 and
R₉ represents a C₁ to C₁₀ alkyl group, or a cyclic ketal group containing 5 to 7 carbon atoms, said cyclic ketal group being unsubstituted or substituted with at least one linear or branched C₂-C₃₀ alkyl, a sterol group, a diacyl glycerol, a hydrofluorocarbon-based chain or at least one monosaccharide or polysaccharide,
Y is an oxygen atom, an NH group or a sulfur atom,
and R" is a hydrocarbon-based chain or a fluorocarbon-based chain,
R' is a hydrocarbon-based chain,
or
• are linked, independently of one another, via a covalent bond to another substituent R₃ or R₃', which may be identical or different, of another compound of formula (I), which may be identical or different, so as to form a compound in dimer form.

4. The use according to claim 1, wherein:
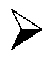 X represents an oxygen atom,
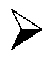 B represents an unnatural pyrimidic base substituted with a heteroaryl group containing three nitrogen atoms, said heteroaryl group being substituted with a group: 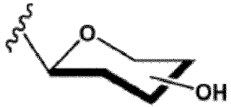
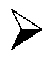 substituents L₁ and L₂ are identical or different and represent:
(i) a hydrogen atom,
(ii) a hydroxyl group,
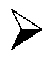 R₃
• represents a heteroaryl group containing three nitrogen atoms, said heteroaryl group being substituted with a group: 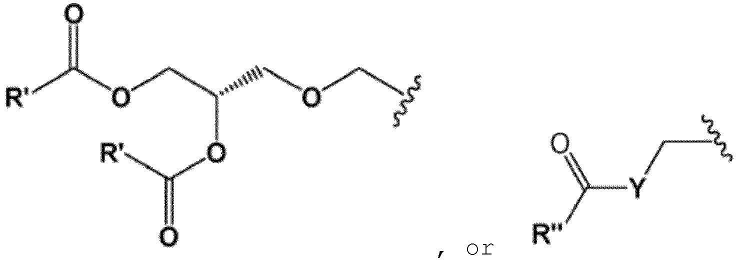 wherein:
R' is a hydrocarbon-based chain;
Y is an oxygen atom, an NH group or a sulfur atom,
and R" is a hydrocarbon-based chain or a fluorocarbon-based chain,
or
• is linked to another substituent R₃, which may be identical or different, of another compound of formula (I), which may be identical or different, so as to form a compound in dimer form.

5. The use according to claim 1, wherein
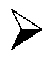 B represents a puric or pyrimidic base;
Y substituents L₁ and L₂ form a ketal group of formula (II) below: 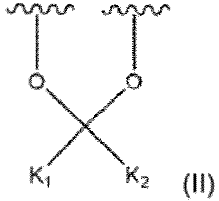 formula (II) in which K₁ and K₂ are identical or different and represent a saturated or unsaturated C₁-C₁₉ hydrocarbon-based chain,
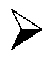 R₃
• represents a phosphocholine group; or
• is linked to another substituent R₃, identical or different, of another compound of formula (I), which may be identical or different, so as to form a compound in dimer form.

6. The use according to claim 1, wherein said low-molecular-weight molecule is chosen from:
- the group defined by formula (III) below, wherein R' is a hydrocarbon-based chain: 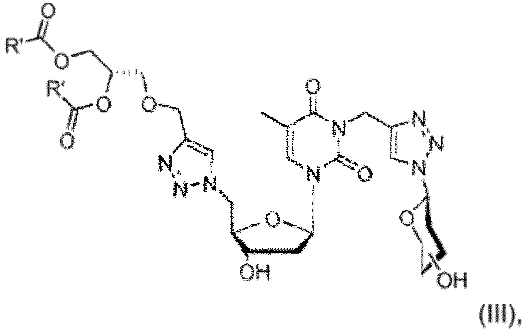
- the group defined by formula (IV) below, wherein Y is an oxygen atom, an NH group or a sulfur atom, and R" is a hydrocarbon-based chain or a fluorocarbon-based chain: 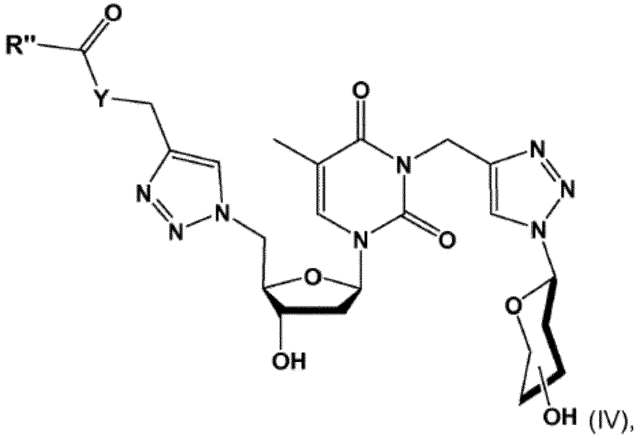
- the group defined by formula (V) below, wherein n is an integer between 0 and 19: 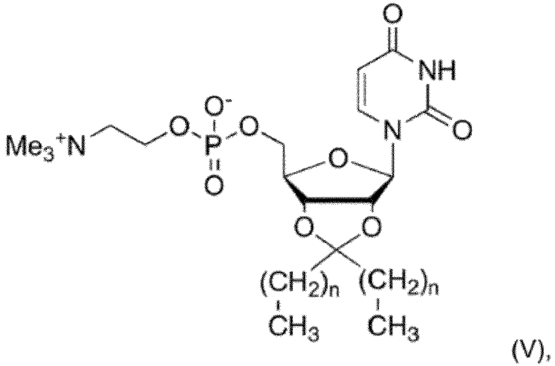 or
- the group defined by formula (VI) below, wherein n is an integer between 0 and 19:

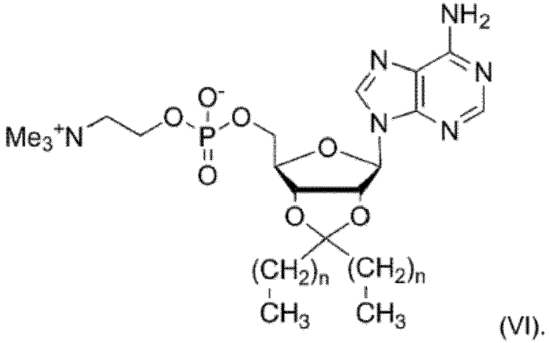

7. The use according to claim 1, wherein said low-molecular-weight molecule is chosen from the group comprising:
- 5'-(4-((2*H*,2*H*,3*H*,3*H*-perfluoroundecanamide)methyl)-1*H-*1,2,3-triazol-1-yl)-N3-(1-((β-D-glucopyranoside)-1H-1,2,3,-triazol-4-yl)methyl)thymidine,
- 5'-(4-((1H,1H,2H,2H-perfluoroundecanamide)methyl)-1H-1,2,3-triazol-1-yl)-N3-(1-((β-D-glucopyranoside)-1H-1,2,3-triazol-4-yl)methyl)thymidine,
- 5'-(4-((oleamide)methyl)-1H-1,2,3-triazol-1-yl)-N3-(1-((β-D-glucopyranoside)-1H-1,2,3-triazol-4-yl)methyl)thymidine,
- 5'-(4-((stearamide)methyl)-1H-1,2,3-triazol-1-yl)-N3-(1-((β-D-glucopyranoside)-1H-1,2,3-triazol-4-yl)methyl)thymidine,
- 5'-(4-((octadecyloxy)methyl)-1H-1,2,3-triazol-1-yl)-N3-(1-((β-D-glucopyranoside)-1H-1,2,3-triazol-4-yl)methyl)thymidine, and
- 5'-(4-((cholesteryloxy)methyl)-1H-1,2,3-triazol-1-yl)-N3-(1-((β-D-glucopyranoside)-1H-1,2,3-triazol-4-yl)methyl)thymidine,
- 2',3'-O-18-pentatriacontanylidenuridine-5'-phosphocholine,
- 2',3'-O-18-pentatriacontanylidenadenosine-5'-phosphocholine, and
a mixture of at least two of these compounds.

8. The use according to anyone of claims 1 to 7, wherein the concentration of supramolecular system used is between 0.001 mg.ml⁻¹ and 100 mg.ml⁻¹ of aqueous medium.

9. The use according to anyone of claims 1 to 8, wherein the particles have a size of between 0.5 nm and 5 µm, advantageously between 5 and 100 nm.

10. The use according to anyone of claims 1 to 9, wherein the liquid medium is water.

11. A process for subtracting nanoparticles from a liquid medium containing same, comprising a step (a) of adding a supramolecular system to said liquid medium at a temperature of between 2 and 95°C, wherein the supramolecular system comprises at least one low-molecular-weight molecule of formula (I) as defined in anyone of claims 1 to 7, said supramolecular system forming a gel on contact with the liquid medium, said gel capturing the nanoparticles contained in said liquid medium, and a step (b) of separating said liquid medium and said gel having captured said nanoparticles.

12. The process according to claim 11, also comprising, before step (b), when the temperature of the liquid medium obtained in step (a) is below 50°C, the following intermediate steps:
(a1) heating the medium obtained in step (a) to a temperature of between 50 and 95°C, and
(a2) cooling the medium obtained in step (a1) to a temperature of between 2 and 50°C.

13. The process according to claim 11 or 12, wherein the concentration of supramolecular system used is between 0.001 mg.ml⁻¹ and 100 mg.ml⁻¹ of aqueous medium.

14. The process according to anyone of claims 11 to 13, wherein the particles have a size of between 0.5 nm and 5 µm, advantageously between 5 nm and 100 nm.

15. The process according to anyone of claims 11 to 14, wherein the liquid medium is water.

16. A group of compounds of formula (IV) below: 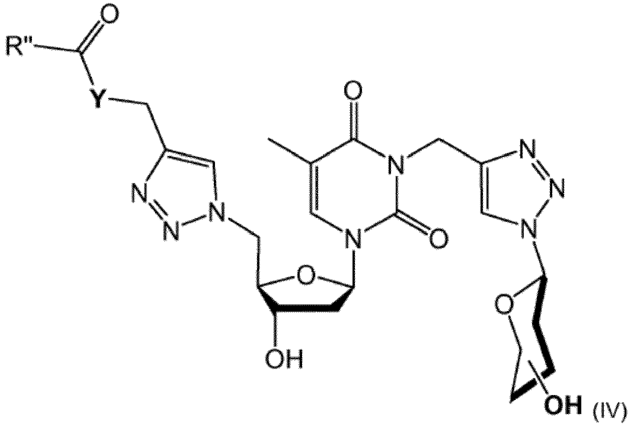 wherein Y is an oxygen atom, and R" is a hydrocarbon-based chain or a fluorocarbon-based chain.
